# EUROPEAN PATENT SPECIFICATION

(11) **EP 3 758 692 B1**
(45) Date of publication and mention of the grant of the patent: **21.08.2024**
(21) Application number: 19760644.5
(22) Date of filing: 27.02.2019
(51) Int. Cl.: A61K 31/17, A61K 31/24, A61K 31/56, A61K 31/64, A61K 45/06, A61K 9/00, A61P 25/00

(54) **COMPOSITIONS AND METHODS FOR TREATING PAIN**
VERBINDUNGEN UND VERFAHREN ZUR BEHANDLUNG VON SCHMERZEN
COMPOSITIONS ET MÉTHODES POUR TRAITER LA DOULEUR

(30) Priority: 27.02.2018 US 201862636118 P
(43) Date of publication of application: 06.01.2021
(73) Proprietor: University of Maryland, Baltimore, Baltimore, MD 21201-1508 (US); The United States Government as represented by The Department of Veterans Affairs, Washington, DC 20420 (US)
(72) Inventor: SIMARD, J., Marc, Baltimore, MD 21212-3421 (US); GERZANICH, Vladimir, Elkridge, MD 21075 (US)
(74) Representative: Schwarz & Partner Patentanwälte GmbH
(86) International application number: PCT/US2019/019852
(87) International publication number: WO 2019/168997

(56) References cited:
- WO-A1-2009/000038
- WO-A1-2018/023035
- CN-A- 105 769 897
- CN-A- 105 884 716
- US-A1- 2009 130 083
- US-A1- 2010 092 469
- US-A1- 2010 316 678
- SALAM OMAR M E ABDEL ET AL: "Effect of hypoglycaemia induced by different agents on thermal Nociception in mice", BULLETIN OF THE NATIONAL RESEARCH CENTER, CAIRO, EG, vol. 32, no. 4, 1 January 2007 (2007-01-01), pages 377 - 392, XP009530422, ISSN: 1110-0591
- BERTOZZI, MM ET AL.: "Diosmin reduces chronic constriction injury-induced neuropathic pain in mice", CHEMICO-BIOLOGICAL INTERACTIONS, vol. 273, 1 August 2017 (2017-08-01), pages 180 - 189, XP085131464, doi:10.1016/j.cbi.2017.06.014

## Description

### FIELD OF THE INVENTION

The field of the invention relates to therapeutics for treating neuropathic pain.

### BACKGROUND OF THE INVENTION

Neuropathic pain can arise from aberrant regeneration of damaged nerves, and is characterized by hyperalgesia and allodynia - increased sensitivity to pain, and exaggerated pain response to normally non-painful stimuli. Ji et al. Nat Rev Drug Discov 2014;13:533-48. Almost any pathological process that damages neural tissues can cause neuropathic pain. Jay et al., Dis Mon 2014;60:6-47.

Neuroinflammation induced by neural damage, and mediated mainly by proinflammatory cytokines and chemokines, plays a critical role in establishing and maintaining neuropathic pain after peripheral nerve injury (PNI). Neuroinflammation is characterized by infiltrating leukocytes, glial cell activation, and production of inflammatory mediators. Both peripheral and central inflammation drive chronic pain. Ji et al. NatRev Drug Discov 2014;13:533-48.

Glial activation is a key mechanism in the genesis of chronic pain. Ji et al., Pain 2013;154 Suppl 1:S10- 28; Mika et al. Eur J Pharmacol 2013;716:106-19. Microglia, astrocytes, and oligodendrocytes modulate CNS inflammation triggered by PNI. Machelska et al. F1000Res 2016;5:2743. Microglia are first responders and play crucial roles early on, whereas astrocytes become involved later. Among the many factors secreted by activated astrocytes, three stand out: IL-6, CCL2 and CXCL1. Oka et al., Brain Res 1995;692:123-8; Deleo et al., J Interferon Cytokine Res 1996;16:695-700; Xu et al., Cytokine 1997;9:1028-33; Malan et al., Neuron Glia Biol 2011;7:117-28; Wei et al., Exp Neurol 2013;241:159-68; Zhou et al., J Neuroinflammation 2016;13:141; Menetski et al., Neuroscience 2007;149:706-14; Zhang et al., J Neurosci 2007;27:12396- 406; Thacker et al., Eur J Pain 2009;13:263- 72; Zhang et al., J Neuroinflammation 2012;9:136; Zhang et al., Pain 2013;154:2185-97; Cao et al., Exp Neurol 2014;261:328-36; Cao et al., Brain Res Bull 2016;127:219-25; Yang et al., Neurosci Lett 2016;626:135-41.

Chemokines such as CCL2 and CXCL1 are best known for recruiting leukocytes, but they also act on their cognate receptors, CCR2 and CXCR2, expressed on dorsal horn neurons (astrocyte-to-neuron signaling), resulting in synaptic hyperexcitability in the dorsal horn that leads to hypersensitivity following PNI. Cao et al., Exp Neurol 2014;261:328-36; Yang et al., Neurosci Lett 2016;626:135-41; Tiwari et al., Neurosci Biobehav Rev 2014;45:19-27; Zhang et al., Cell Mol Life Sci 2017 PMID: 28389721. In neuropathic pain models, blockade or gene deletion of each of these individually results in significant attenuation of pain-related behaviors. Targeting all three simultaneously, without disturbing the beneficial roles of astrocytes, is a key goal.

WO 2009/000038 discloses a combination of agents to treat neuropathic pain. According to WO 2009/000038 neurokinin (NK) antagonists shall be administered in combination with other compounds, including sodium channel blockers, local anaesthetics, modulators of TRPVl receptors, NMDA-receptor antagonists, calcium channel antagonists, opioids and modulators of CB2 receptors, neurosteroids, alpha 2 adrenoceptor agonists, and non-steroidal anti-inflammatory (NSAIDS).

Evidence-based recommendations for treating neuropathic pain include opioids, which remain the most consistently effective class of drugs for this condition. In many cases, previously opioid-naive patients are prescribed opioids for neuropathic pain. However, in light of the opioid epidemic, recent guidelines from the Centers for Disease Control and Prevention advise against the routine use of µ-opioid receptor-selective agents. According to the Surgeon General of the USA, 78 people die every day from opioid overdose, demanding urgent solutions for this public health crisis. Using non-addicting drugs to target non-opioid pathways involved in the central mechanisms of neuropathic pain has the potential to dramatically impact this epidemic of opioid addiction and overdose. Thus, there is an urgent need for new compositions and methods for treating neuropathic pain in subjects.

The foregoing description of the background is provided to aid in understanding the invention, and is not admitted to be or to describe prior art to the invention.

### SUMMARY OF THE INVENTION

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention.

Peripheral nerve injury due to numerous causes is a major risk factor for the development of neuropathic pain, which can persist indefinitely, can severely impact quality-of-life, and can lead to opioid use that predisposes to addiction. Described herein are investigations on the role of astrocytic NF-κB signaling and Sur1-Trpm4 in signs of chronic pain in a murine model of peripheral nerve injury.

In one aspect, the present invention provides an agent that inhibits the activity of an NC_{Ca-ATP} channel for the use of treating neuropathic pain in a subject in need thereof. The agent is a) a SUR1 antagonist; or b) a Transient Receptor Potential cation channel subfamily M member 4 (TRPM4) antagonist.

The SUR1 antagonist is selected from the group consisting of glibenclamide (glyburide), tolbutamide, acetohexamide, chlorpropamide, tolazimide, glipizide, gliquidone, repaglinide, nateglinide, meglitinide, gliclazide, glimepiride, repaglinide, nateglinide and mitiglinide.

The TRPM4 antagonist is selected from flufenamic acid, mefanimic acid, and niflumic acid.

### BRIEF DESCRIPTION OF THE FIGURES

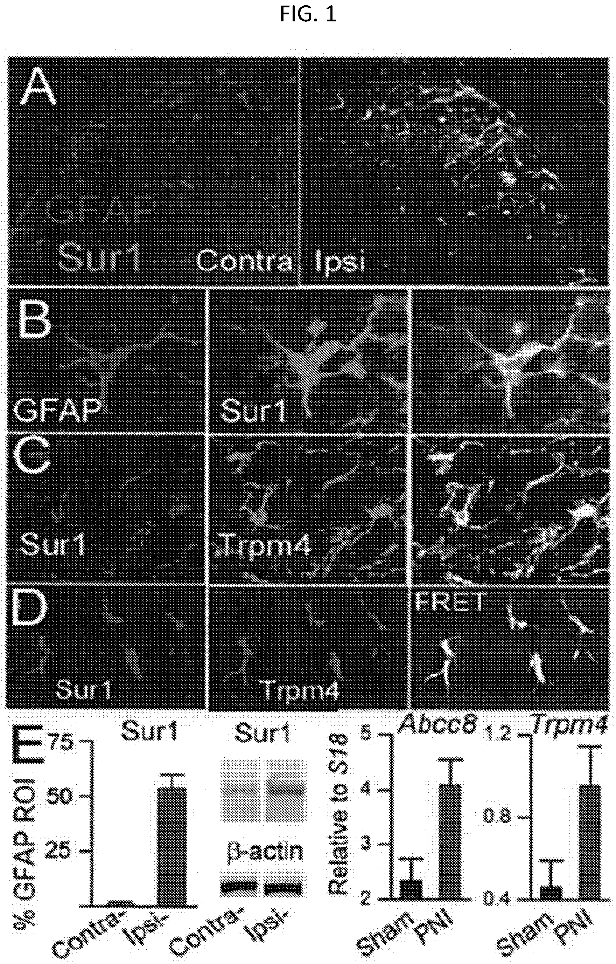

FIG. 1. Sur1-Trpm4 upregulation in dorsal horn astrocytes after sciatic n. cuffing. A, B: Double immunolabeling for GFAP (red) plus Sur1 at low (A) and high (B) magnification, ipsilateral (PNI) and contralateral (CTR) to PNI; superimposed images in A and B, right. C, D: Double immunolabeling for Sur1 plus Trpm4, ipsilateral to PNI; superimposed (C, *right*) and FRET (D, *right*) images also shown. E: Quantification of Sur1 in the GFAP+ ROI (left), confirmed by immunoblot (*middle*) and by qPCR (*right*); mean ± SE; 5/group.

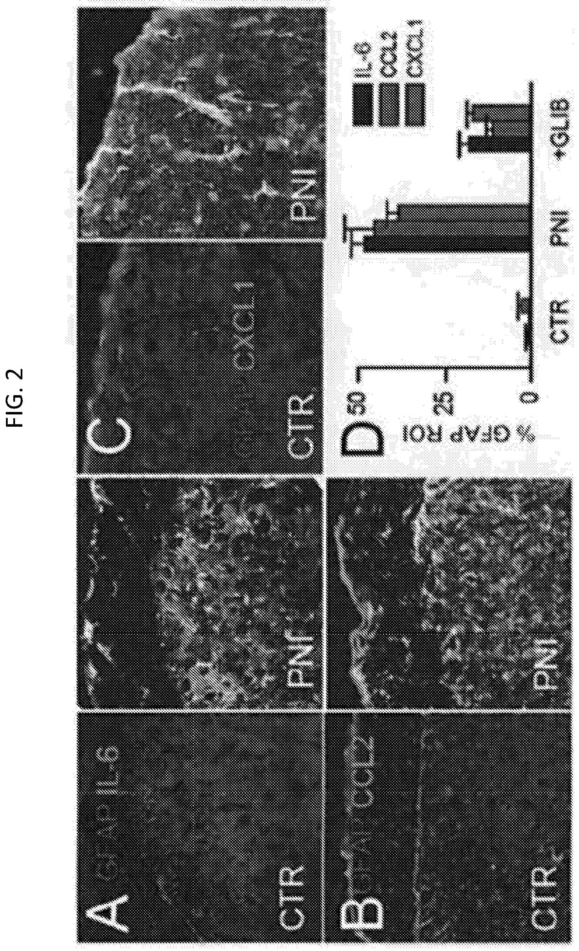

FIG. 2. IL6, CCL2, CXCL1 upregulation in dorsal horn after sciatic n. cuffing. A-C: Double immunolabeling for GFAP (red) plus IL-6 (A), CCL2 (B) and CXCL1 (C), ipsilateral (PNI) and contralateral (CTR) to PNI, in untreated WT mice. D: Quantification in the GFAP+ROI for IL-6, CCL2 and CXCL1, in untreated PNI mice and PNI mice treated with glibenclamide for 14 days; mean ± SE; 5/group.

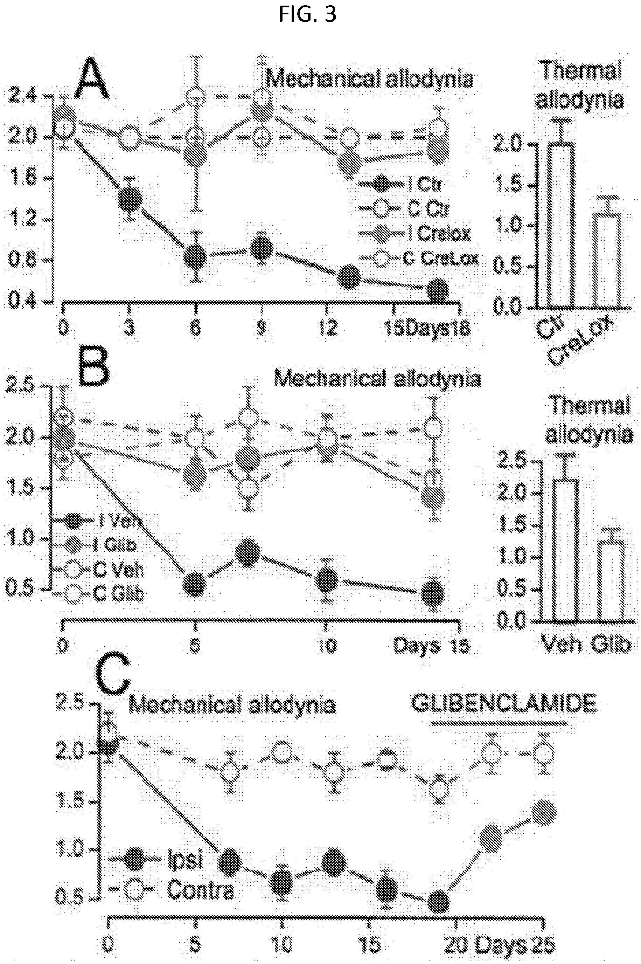

FIG. 3. Astrocyte-specific *Abcc8*-KO and glibenclamide-treated mice are protected from allodynia after sciatic n. cuffing. A, B: Mechanical (*left*) and cold (*right*) allodynia in ipsilateral (I) and contralateral (C) hindpaws of *Abcc8*^{loxP/loxP}/Gfap-Cre mice and littermate Cre controls (A), and WT mice treated with glibenclamide x14 days (B); 5/group. C: Mechanical allodynia in WT mice treated with glibenclamide beginning on day 18 after PNI; mean± SE; 5/group.

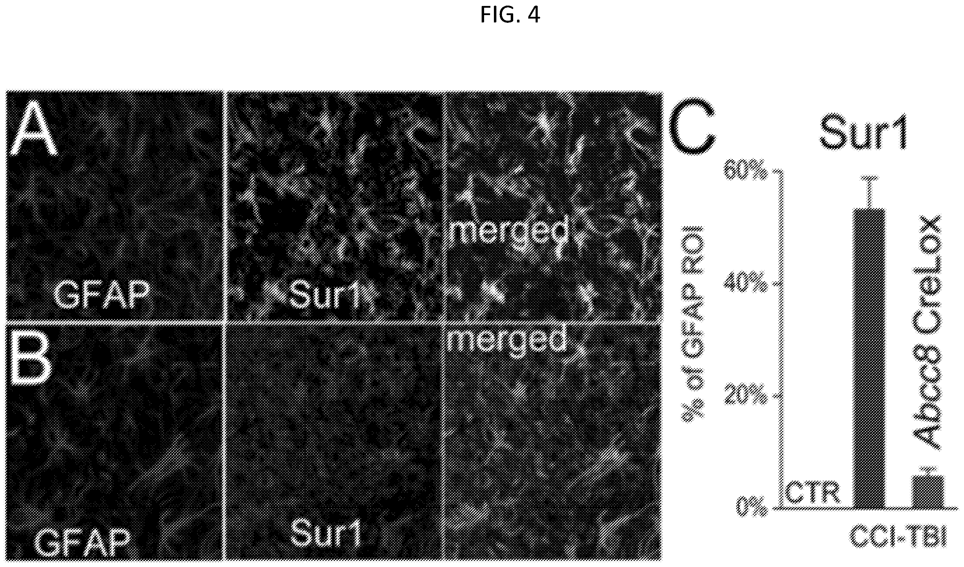

FIG. 4. Astrocyte-specific deletion of *Abcc8* prevents expression of Sur1 by astrocytes 10-days post-injury (B, C), compared to WT (A); n=3/group. CTR=control; CCI-TBI=traumatic brain injury induced by controlled cortical impact.

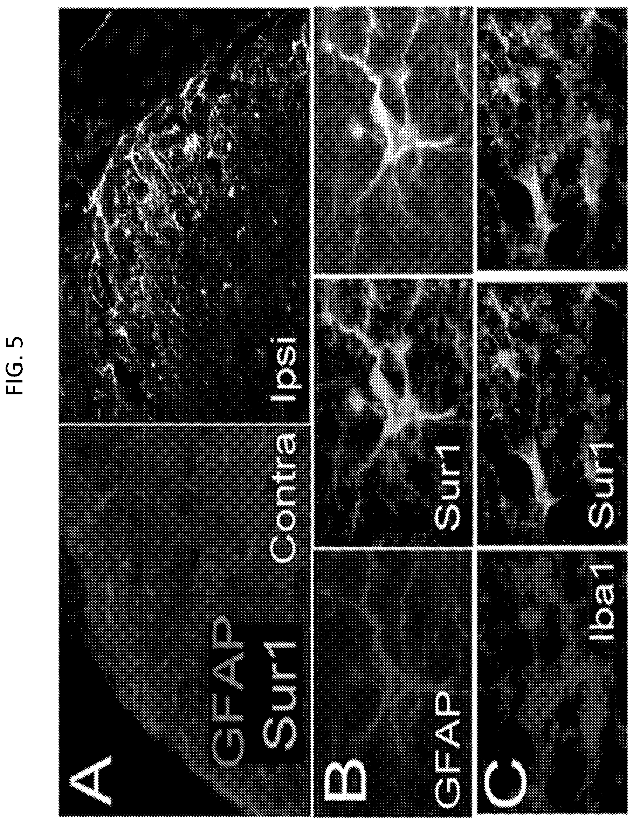

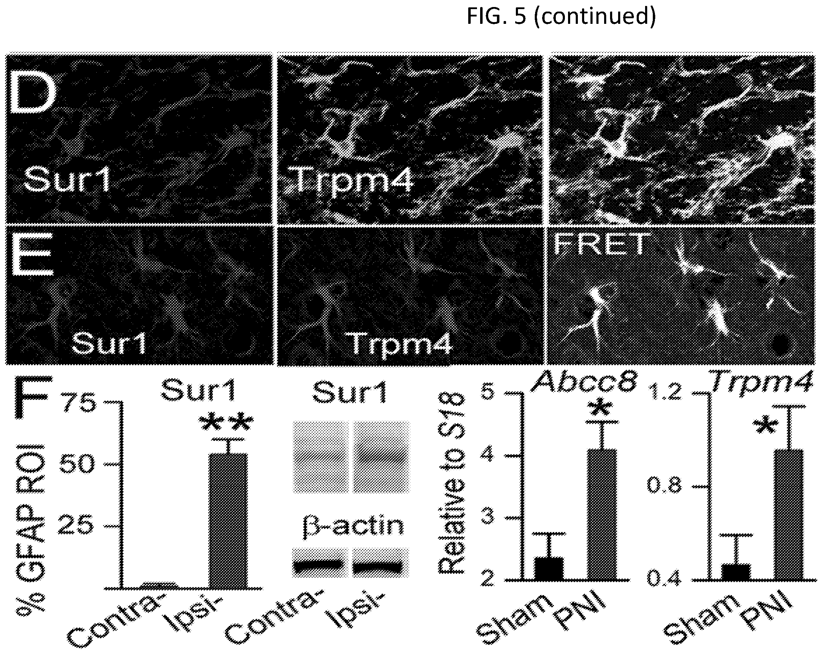

FIG. 5. SUR1-TRPM4 upregulation in dorsal horn astrocytes after sciatic n. cuffing. A,B: Double immunolabeling for GFAP plus SUR1 at low (A) and high (B) magnification, ipsilateral (PNI) and contralateral (CTR) to PNI; superimposed images in A and B, right. C: Double immunolabeling for SUR1 plus Iba1 shows minimal overlap. D,E: Double immunolabeling for SUR1 plus TRPM4, ipsilateral to PNI; superimposed (D, right) and FRET (E, right) images also shown. F: Quantification of SUR1 in the GFAP+ ROI (left), confirmed by immunoblot (middle) and by qPCR (right); mean ± SE; 5/group; *, P<0.05; **, P<0.01.

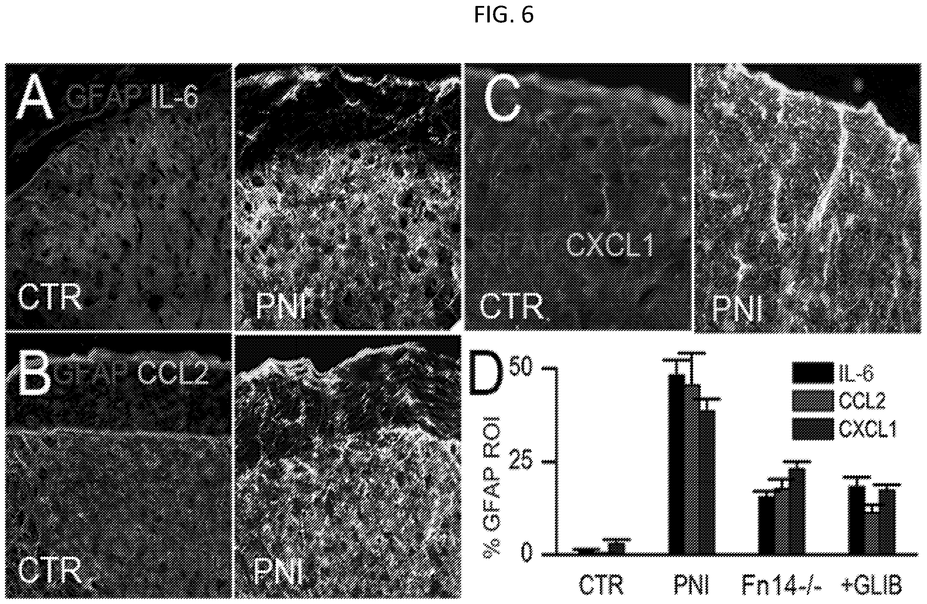

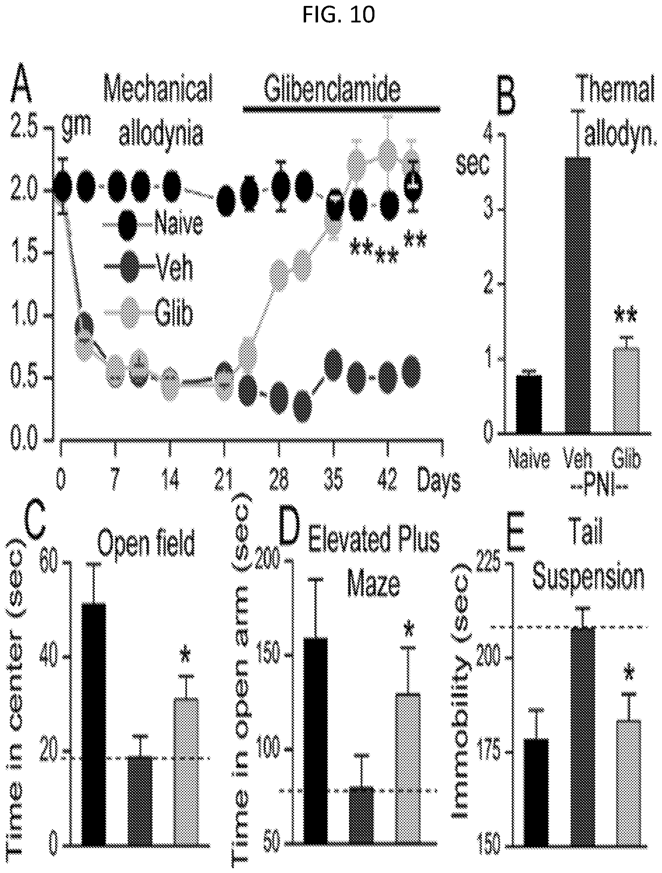

FIG. 6. IL6, CCL2, CXCL1 upregulation in dorsal horn after sciatic n. cuffing. A-C: Double immunolabeling for GFAP plus IL-6 (A), CCL2 (B) or CXCL1 (C), ipsilateral (PNI) and contralateral (CTR) to PNI, in untreated WT mice. D: Quantification in the GFAP+ region of interest (ROI) for IL-6, CCL2 and CXCL1, in untreated WT (PNI) mice and WT mice treated with glibenclamide, (tissues from experiment in Fig. 10); mean ± SE; 5/group; **, *P*<0.01.

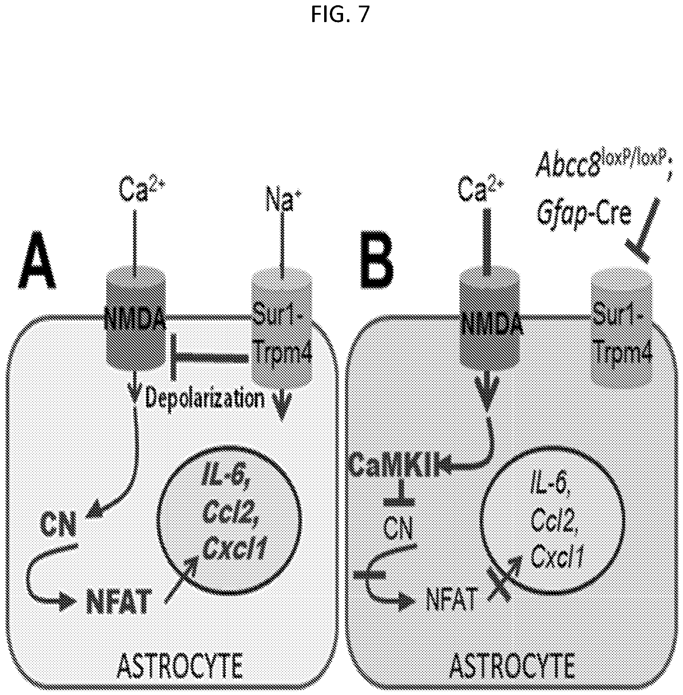

FIG. 7. Normally, Ca²⁺ influx is sufficient to activate CN (but not CaMKII), resulting in NFAT nuclear translocation (A). Inhibition of Sur1-Trpm4 increases [Ca²⁺]ᵢ, resulting in activation, which inhibits CN, reducing NFAT nuclear translocation (B).

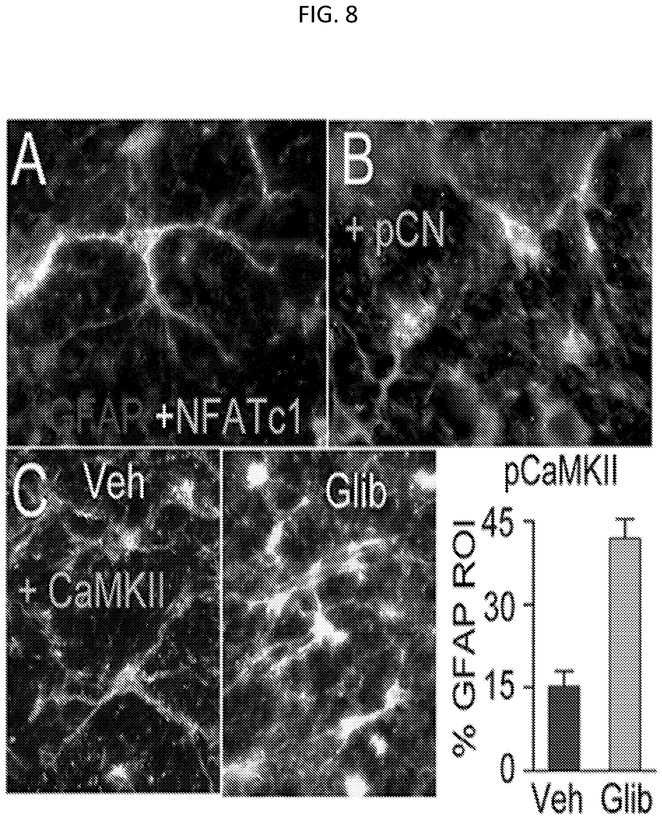

FIG. 8. Astrocytes post-PNI express NFATc1 and pCN (A,B), and minimal pCaMKII (C, veh); treatment with glibenclamide increases astrocytic pCaMKII (C, glib, D); mean ± SE; n=3/group.

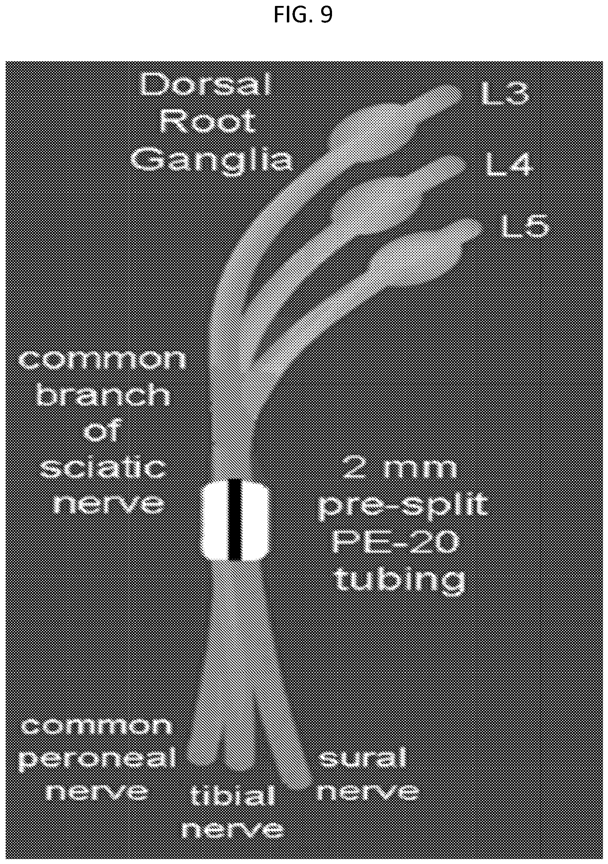

FIG. 9. Sciatic nerve cuff model of neuropathic pain.
FIG. 10. Glibenclamide gradually reverses allodynia and other pain behaviors. A-E: The sciatic n. of WT mice was cuffed and, beginning on day21, mice were administered vehicle (Veh) or glibenclamide, 10 µg IP once daily (Glib); behaviors were compared to those of uninjured littermates (naive). Mechanical (A) and cold (B) allodynia, and anxiety- and depression-like behaviors (C-E) were worsened by PNI and gradually ameliorated by glibenclamide; 6 mice/group; *, P<0.05; **, P<0.01.

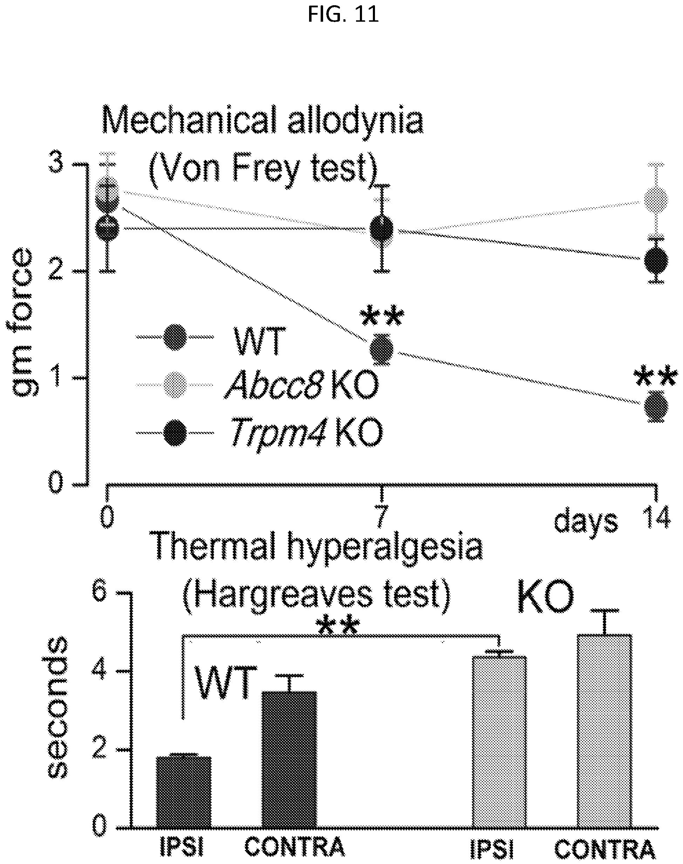

FIG. 11. Deletion of *Abcc8* or of *Trpm4* recapitulates protective effect of glibenclamide on neuropathic pain behaviors. Compared to wild-type (WT) mice, knock-out (KO) mice failed to develop mechanical allodynia or thermal hyperalgesia after sciatic n. cuffing; 3 mice/group.

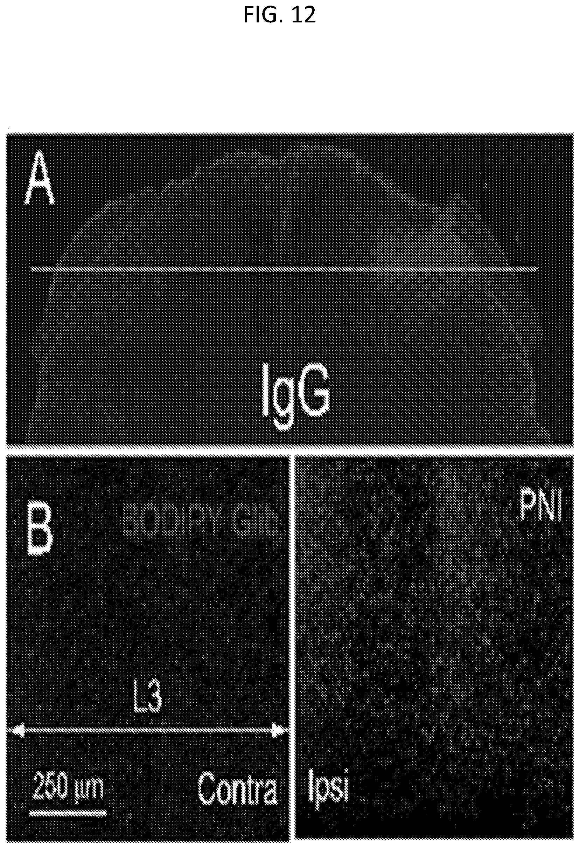

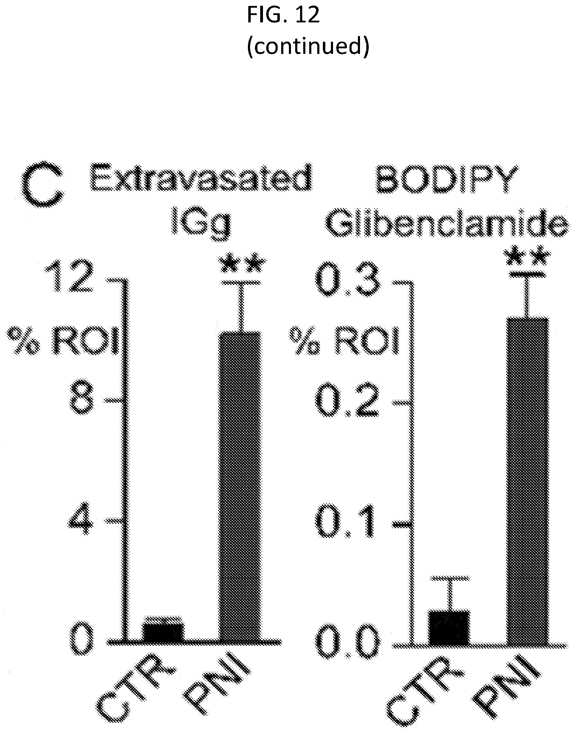

FIG. 12. A: After sciatic n. cuffing, BBB leakage is shown by IgG extravasation in ipsilateral dorsal horn. B: Fluorescent bodipy-glibenclamide enters ipsilateral (PNI) but not contralateral (Contra) dorsal horn at the level indicated by line in (A). C: Quantification for extravasated IgG and bodipy-glibenclamide in the dorsal horn; 3/group; **, P<0.01.

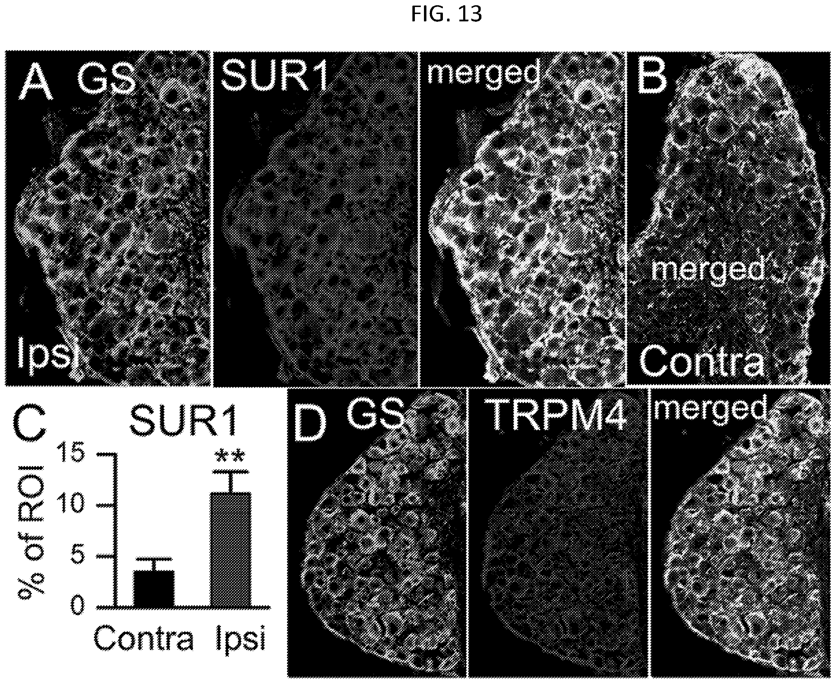

FIG. 13. SUR1-TRPM4 is upregulated in DRG satellite cells after sciatic n. cuffing. A,B: Co-immunolabeling for glutamine synthase (GS) and SUR1 in ipsilateral (A) and contralateral (B) DRG. C: quantification of SUR1 in contra- and ipsi-lateral DRG; n=5/group; **, P<0.01. D: Co-immunolabeling for GS and TRPM4 in ipsilateral DRG.

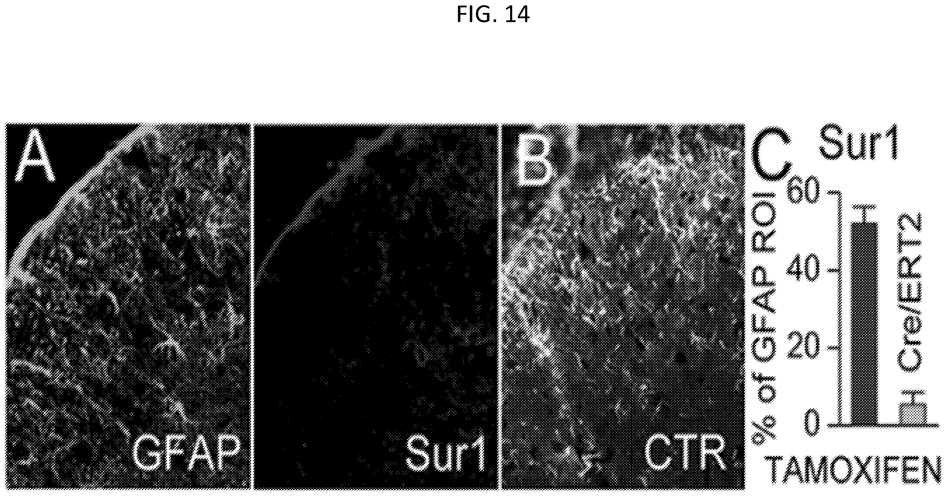

FIG. 14. *Abcc8*^{loxP/loxP}/*GFAP*-Cre/ERT2. GFAP-driven deletion of *Abcc8* induced by tamoxifen reduces expression of SUR1 by dorsal horn astrocytes 10-days post-injury (A), compared to CTR (B). C: Quantification; n=3/group.

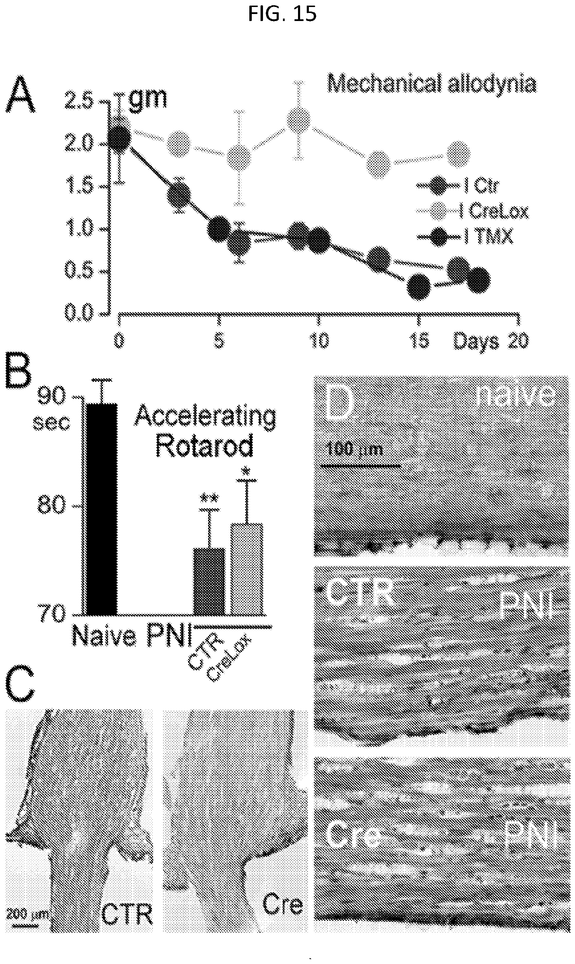

FIG. 15. A: Mechanical allodynia after n. cuffing in ipsilateral hindpaws of *Abcc8*^{loxP/loxP}/GFAP-Cre/ERT2 mice (Cre, green), littermate Cre/ERT2 controls (CTR, red), and WT mice treated with tamoxifen TMX, blue; mean ± SE; 5/group; **, *P*<0.01. B: Time on accelerating rotarod for naive WT mice and, after PNI, for *Abcc8*^{loxP/loxP}/GFAP-Cre/ERT2, and littermate controls (CTR); mean ± SE; 5/group; *, *P*<0.05; **, *P*<0.01. C,D: H&E sections of cuffed n. at low (C) and high (D) resolution, compared to uninjured n. (naive).

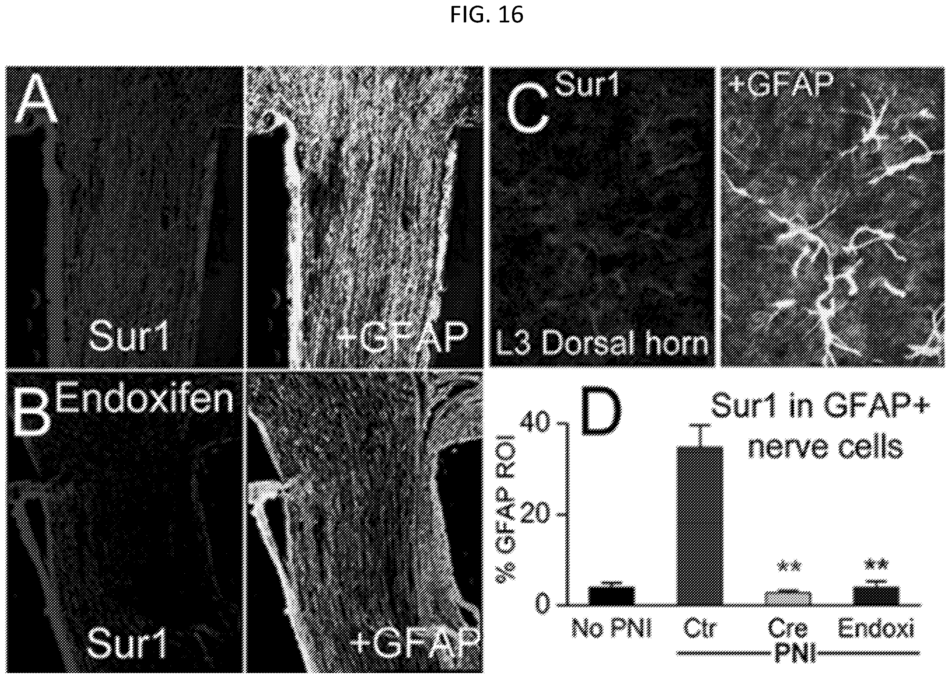

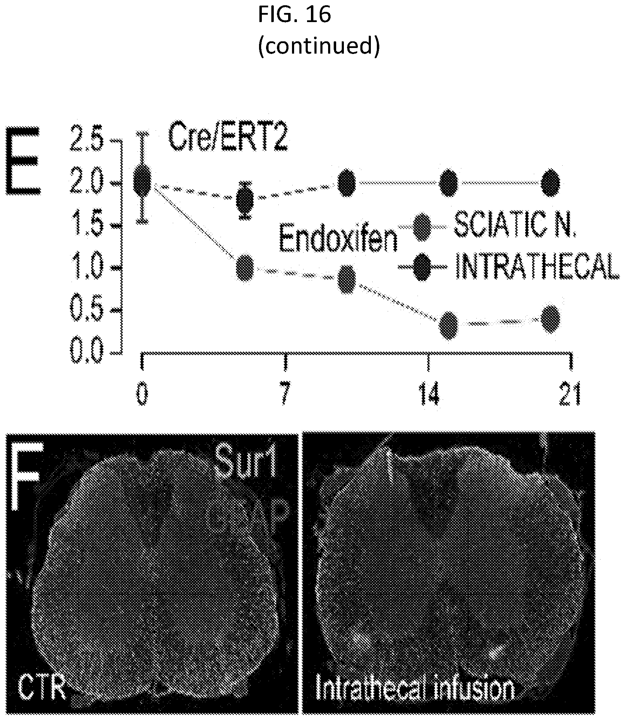

FIG. 16. A,B: After PNI, SUR1 is expressed by GFAP+ glia in nerve of WT (A) but not *Abcc8*^{loxP/loxP}/*GFAP*-Cre/ERT2 mice given endoxifen on the nerve (B) before cuffing. C: Endoxifen applied to the nerve does not prevent SUR1 upregulation by dorsal horn astrocytes. D: Quantification of SUR1 in nerves from *Abcc8*^{loxP/loxP}/*GFAP*-Cre mice (Cre), *Abcc8*^{loxP/loxP}/*GFAP*-Cre/ERT2 (Endoxi); 5/group; **, *P*<0.01. E: Allodynia is prevented by administration of endoxifen intrathecally (blue) but not on the nerve (red); 3/group. F: Intrathecal infusion of glibenclamide in an uninjured, naive mouse does not upregulate GFAP or SUR1.

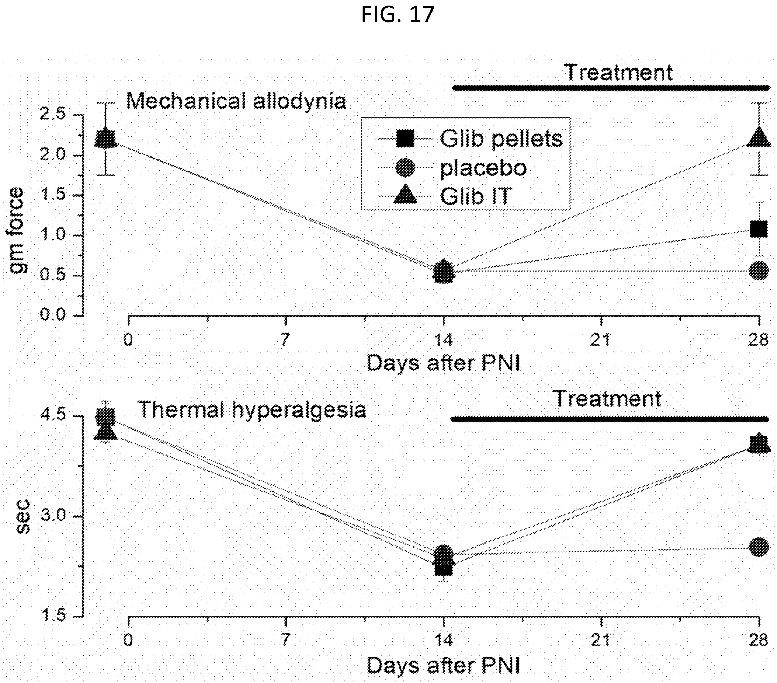

FIG. 17. Various formulations and routes of administration of glibenclamide are effective at ameliorating neuropathic pain behaviors following peripheral nerve injury.

### DETAILED DESCRIPTION OF THE INVENTION

The ongoing opioid epidemic is driven in part by legitimate use of opioids prescribed for neuropathic pain. Targeting alternative pathways for pain control using non-addicting drugs is a major goal of neuropathic pain research. After peripheral nerve injury (PNI), cytokines and chemokines are upregulated centrally, where they contribute mechanistically to the pathogenesis of neuropathic pain. Reactive astrocytes in the dorsal horn exhibit a chronically activated, pro-inflammatory secretory (CAPS) phenotype characterized by secretion of cytokines, chemokines, and other factors, including interleukin-6 (IL-6), chemokine C-C motif ligand 2 (CCL2) and chemokine C-X-C motif ligand 1 (CXCL1). The post-PNI astrocytic CAPS phenotype contributes not only to inflammation but also to neuronal hyperactivation via neuronal chemokine receptors, leading to neuropathic pain.

Herein, we demonstrate that, in a murine model of neuropathic pain induced by sciatic nerve cuffing, Sur1-Trpm4 channels are newly upregulated in dorsal horn astrocytes, and that inhibition of astroglial Sur1-Trpm4 reduces the expression of IL-6, CCL2 and CXCL1, and significantly ameliorates neuropathic pain.

Reference will now be made in detail to embodiments of the invention which, together with the drawings and the following examples, serve to explain the principles of the invention. These embodiments describe in sufficient detail to enable those skilled in the art to practice the invention, which is defined by the claims. Unless defined otherwise, all technical and scientific terms used herein have the same meanings as commonly understood by one of ordinary skill in the art.

For the purpose of interpreting this specification, the following definitions will apply and whenever appropriate, terms used in the singular will also include the plural and vice versa. In the event that any definition set forth below conflicts with the usage of that word in any other document, the definition set forth below shall always control for purposes of interpreting this specification and its associated claims unless a contrary meaning is clearly intended (for example in the document where the term is originally used). The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. Furthermore, where the description of one or more embodiments uses the term "comprising," those skilled in the art would understand that, in some specific instances, the embodiment or embodiments can be alternatively described using the language "consisting essentially of" and/or "consisting of." As used herein, the term "about" means at most plus or minus 10% of the numerical value of the number with which it is being used.

One skilled in the art may refer to general reference texts for detailed descriptions of known techniques discussed herein or equivalent techniques. These texts include Current Protocols in Molecular Biology (Ausubel et. al., eds. John Wiley & Sons, N.Y. and supplements thereto), Current Protocols in Immunology (Coligan et al., eds., John Wiley St Sons, N.Y. and supplements thereto), Current Protocols in Pharmacology (Enna et al., eds. John Wiley & Sons, N.Y. and supplements thereto) and Remington: The Science and Practice of Pharmacy (Lippincott Williams & Wilicins, 2Vt edition (2005)), for example.

The invention is based on the surprising discovery that Sur1-Trpm4 channels are upregulated in dorsal horn astrocytes in a model of neuropathic pain, and that inhibition of astroglial Sur1-Trpm4 reduces the expression of IL-6, CCL2 and CXCL1, and significantly ameliorates neuropathic pain.

As used herein, the term "NC_{Ca-ATP} channel" refers to a non-selective cation channel complex that is activated by intracellular calcium and blocked by intracellular ATP, and has a single-channel conductance to potassium ion (K⁺) of between about 20 and about 50 pS at physiological potassium concentrations. This channel complex includes a SUR1 receptor and is sensitive to SUR1 agonists and antagonists. The channel complex may include a pore that has similar properties to the TRPM4 channels, including blockade by TRPM4 blockers (such as, e.g., flufenamic acid, mefanimic acid, and niflumic acid), and therefore the pore of the NC_{Ca-ATP} channel complex is TRPM4 channel. This channel complex is referred to herein as a "channel" and is described in greater detail elsewhere in the application.

As used herein, the term "TRPM4 channel" refers to a pore that passed ions that is a member of the transient receptor potential channel family (hence the acroym "TRP") and is the pore forming portion of the SUR1-sensitive NC_{Ca-ATP} channel.

As used herein, the term "antagonist" refers to a biological or chemical agent that acts within the body to reduce the physiological activity of another chemical or biological substance. In the present invention, the antagonist blocks, inhibits, reduces and/or decreases the activity of a NC_{Ca-ATP} channel of a cell, such as an astroglial cell. Without being bound by theory, the antagonist combines, binds, associates with a NC_{Ca-ATP} channel of an astroglial cell such that the NC_{Ca-ATP} channel is closed (deactivated), meaning reduced biological activity with respect to the biological activity in the diseased state. The antagonist combines, binds and/or associates with a regulatory subunit of the NC_{Ca-ATP} channel, particularly SUR1, or the antagonist combines, binds, and/or associates with a pore-forming subunit of the NC_{Ca-ATP} channel, such that the NC_{Ca-ATP} channel is closed (deactivated). The terms antagonist or inhibitor can be used interchangeably.

As used herein, the terms "effective amount" or "therapeutically effective amount" are interchangeable and refer to an amount that results in an improvement or remediation of at least one symptom of the disease or condition. Those of skill in the art understand that the effective amount may improve the patient's or subject's condition, but may not be a complete cure of the disease and/or condition.

As used herein, the term "inhibit" refers to the ability of the compound to block, partially block, interfere, decrease, reduce or deactivate a channel such as the NC_{Ca-ATP} channel. Thus, one of skill in the art understands that the term inhibit encompasses a complete and/or partial loss of activity of a channel, such as the NC_{Ca-ATP} channel. Channel activity may be inhibited by channel block (occlusion or closure of the pore region, preventing ionic current flow through the channel), by changes in an opening rate or in the mean open time, changes in a closing rate or in the mean closed time, or by other means. For example, a complete and/or partial loss of activity of the NC_{Ca-ATP} channel as may be indicated by a reduction in cell depolarization, reduction in sodium ion influx or any other monovalent ion influx, reduction in an influx of water, reduction in extravasation of blood, reduction in cell death, as well as an improvement in cellular survival following an ischemic challenge.

The term "preventing" as used herein refers to minimizing, reducing or suppressing the risk of developing a disease state or parameters relating to the disease state or progression or other abnormal or deleterious conditions.

The terms "treating" and "treatment" as used herein refer to administering to a subject a therapeutically effective amount of a composition so that the subject has an improvement in the disease or condition. The improvement is any observable or measurable improvement. Thus, one of skill in the art realizes that a treatment may improve the patient's condition, but may not be a complete cure of the disease. Treating may also comprise treating subjects at risk of developing a disease and/or condition.

The NC_{Ca-ATP} channel is distinguished by certain functional characteristics, the combination of which distinguishes it from other ion channels. The characteristics that distinguish the NC_{Ca-ATP} channel include, but are not necessarily limited to, the following: 1) it is a non-selective cation channel that readily allows passage of Na, K, and other monovalent cations; 2) it is activated by an increase in intracellular calcium, and/or by a decrease in intracellular ATP; 3) it is regulated by sulfonylurea receptor type 1 (SUR1, which previously had been considered to be associated exclusively with K_{ATP} channels such as those found in pancreatic β-cells, for example.

The NC_{Ca-ATP} channel was identified first in native reactive astrocytes (NRAs) and later in neurons and capillary endothelial cells after stroke or traumatic brain or spinal cord injury (see at least International Application WO 03/079987). As with the K_{ATP} channel in pancreatic β-cells, the NC_{Ca-ATP} channel is considered to be a heteromultimer structure comprised of sulfonylurea receptor type 1 (SUR1) regulatory subunits and pore-forming subunits. The pore-forming subunits have been characterized biophysically and have been identified as TRPM4.

More specifically, the NC_{Ca-ATP} channel has a single-channel conductance to potassium ion (K⁺) between 20 and 50 pS. The NC_{Ca-ATP} channel is also stimulated by Ca²⁺ on the cytoplasmic side of the cell membrane in a physiological concentration range, where said concentration range is from 10⁻⁸ to 10⁻⁵ M. The NC_{Ca-ATP} channel is also inhibited by cytoplasmic ATP in a physiological concentration range, where said concentration range is from about 10⁻¹ to about 5 mM. The NC_{Ca-ATP} channel is also permeable to the following cations; K⁺, Cs⁺, Li⁺, Na⁺; to the extent that the permeability ratio between any two of said cations is greater than 0.5 and less than 2. The NC_{Ca-ATP} channel is disclosed in U.S. Patent No. 9,375,438.

Disclosed are methods and compositions for treating and/or preventing pain, such as neuropathic pain in a subject. An individual with neuropathic pain or at risk for having neuropathic pain is administered one or more inhibitors of a SUR1-regulated NC_{Ca-ATP} channel. Exemplary inhibitors include sulfonylurea compounds and/or TRPM4-inhibitory compounds, although others are also suitable.

The agent that inhibits the activity of an NC_{Ca-ATP} channel can be administered in a variety of ways and is not particularly limiting. In some embodiments, the agent is administered directly (topically), intravenously, subcutaneously, transcutaneously, intrathecally, intramuscularly, intracutaneously, intragastrically or orally.

Examples of such compounds include an inhibitor of the channel, such as, for example, an antagonist of a type 1 sulfonylurea receptor, such as sulfonylureas like glibenclamide and tolbutamide, as well as other insulin secretagogues such as repaglinide, nateglinide, meglitinide, Mitiglinide, iptakalim, endosulfines, LY397364, LY389382, gliclazide, glimepiride, MgADP, and combinations thereof.

Other such channel inhibitors for the use of the invention are TRPM4 inhibitors flufenamic acid, mefanimic acid, and niflumic acid.

The invention provides a composition for the use of treating neuropathic pain in a subject, comprising an effective amount of an agent that inhibits the NC_{Ca-ATP} channel and a pharmaceutically acceptable carrier.

The agent that inhibits the NC_{Ca-ATP} channel comprises at least an antagonist of a NC_{Ca-ATP} channel of a cell.

The NC_{Ca-ATP} channel is blocked, inhibited, or otherwise is decreased in activity. In such examples, an antagonist of the NC_{Ca-ATP} channel is administered and/or applied. The antagonist modulates the NC_{Ca-ATP} channel such that flux through the channel is reduced, ceased, decreased and/or stopped. The antagonist may have a reversible or an irreversible activity with respect to the activity of the NC_{Ca-ATP} channel of a cell. The antagonist may prevent or lessen the depolarization of the cells thereby lessening cell swelling due to osmotic changes that can result from depolarization of the cells.

Subjects that can be treated with the therapeutic composition of the present invention include subjects suffering from neuropathic pain. Neuropathic pain can be caused by a number of conditions, including, but not limited to alcoholism, amputation, chemotherapy, diabetes, facial nerve problems, HIV infection or AIDS, multiple myeloma, multiple sclerosis, nerve or spinal cord compression from herniated discs or from arthritis in the spine, shingles, traumatic nerve damage or injury, spine surgery, syphilis, thyroid problems, and vitamin B deficiency.

A variety of antagonists to SUR1 are suitable for blocking the channel. Suitable SUR1 antagonists for use according to the invention are selected from the group consisting of glibenclamide (glyburide), tolbutamide, acetohexamide, chlorpropamide, tolazimide, glipizide, gliquidone, repaglinide, nateglinide, meglitinide, gliclazide, glimepiride, repaglinide, nateglinide and mitiglinide.

An effective amount of the inhibitor of the NC_{Ca-ATP} channel that is administered may include a dose of about 0.0001 nM to about 2000 µM. In some embodiments, amount administered is from about 0.01 nM to about 2000 µM; about 0.01 µM to about 0.05 µM; about 0.05 µM to about 1.0 µM; about 1.0 µM to about 1.5 µM; about 1.5 µM to about 2.0 µM; about 2.0 µM to about 3.0 µM; about 3.0 µM to about 4.0 µM; about 4.0 µM to about 5.0 µM; about 5.0 µM to about 10 µM; about 10 µM to about 50 µM; about 50 µM to about 100 µM; about 100 µM to about 200 µM; about 200 µM to about 300 µM; about 300 µM to about 500 µM; about 500 µM to about 1000 µM; about 1000 µM to about 1500 µM; and about 1500 µM to about 2000 µM. Any amount in-between these points is also expected to be of use in the invention.

The inhibitor can be administered parenterally or alimentary. Parenteral administrations include intravenously, intramuscularly, subcutaneous, or intraperitoneally. See, e.g., U.S. Pat. Nos. 6,613,308, 5,466,468, 5,543,158; 5,641,515; and 5,399,363. Alimentary administrations include orally.

The administration of the therapeutic compounds and/or the therapies of the present invention may include systemic, local and/or regional administrations, for example, topically (dermally, transdermally), via catheters, implantable pumps, dermal patches, transdermal patches, etc. Alternatively, other routes of administration are also contemplated such as, for example, arterial perfusion, intracavitary, intraperitoneal, intrapleural, intraventricular and/or intrathecal. The skilled artisan is aware of determining the appropriate administration route using standard methods and procedures. Other routes of administration are discussed elsewhere in the specification.

Treatment methods involve treating an individual with an effective amount of a composition containing an inhibitor of NC_{Ca-ATP} channel or related-compound thereof. An effective amount is described, generally, as that amount sufficient to detectably and repeatedly to ameliorate, reduce, minimize or limit the extent of a disease or its symptoms. The effective amount of an antagonist of NC_{Ca-ATP} channel or related-compounds thereof to be used are those amounts effective to produce beneficial results, particularly with respect to amelioration of pain in the recipient animal or patient.

As is well known in the art, a specific dose level of active compounds such as an antagonist of the NC_{Ca-ATP} channel or related-compounds thereof for any particular patient depends upon a variety of factors including the activity of the specific compound employed, the age, body weight, general health, sex, diet, time of administration, route of administration, rate of excretion, drug combination, and the severity of the particular disease undergoing therapy.

The compound(s) or composition(s) can be administered to the subject once, such as by a single injection or deposition at or near the site of interest. The compound(s) or composition(s) can be administered to a subject over a period of days, weeks, months or even years. In some embodiments, the compound(s) or composition(s) is administered at least once a day to a subject. Where a dosage regimen comprises multiple administrations, it is understood that the effective amount of the compound(s) or composition(s) administered to the subject can comprise the total amount of the compound(s) or composition(s) administered over the entire dosage regimen.

### Inhibitors of NC_{Ca-ATP} Channel

A variety of antagonists to SUR1 are suitable for blocking the channel. Examples of suitable SUR1 antagonists include, but are not limited to glibenclamide, tolbutamide, repaglinide, nateglinide, meglitinide, Mitiglinide, gliclazide, glimepiride, and combinations thereof. In some embodiments of the invention the SUR1 antagonists are selected from the group consisting of glibenclamide and tolbutamide.

The present invention comprises modulators of the channel, for example one or more agonists and/or one or more antagonists of the channel. Examples of antagonists or agonists may encompass respective antagonists and/or agonists identified in U.S. Patent Application Publication No. 2003/0215889.

Antagonists to sulfonylurea receptor-1 (SUR1) are suitable for blocking the channel. Examples of suitable SUR1 antagonists include, but are not limited to glibenclamide, tolbutamide, repaglinide, nateglinide, meglitinide, Mitiglinide, glyclazine, glimepiride, and combinations thereof. In a preferred embodiment of the invention the SUR1 antagonists is selected from the group consisting of glibenclamide and tolbutamide.

TRPM4 inhibitors are suitable for blocking the channel. Suitable TRPM4 inhibitors include, for example, flufenamic acid, mefanimic acid, and niflumic acid.

The agent can comprise a compound (protein, nucleic acid, siRNA, etc.) that modulates transcription and/or translation of SUR1 (regulatory subunit), TRPM4, and/or the molecular entities that comprise the pore-forming subunit.

Transcription factors are regulatory proteins that binds to a specific DNA sequence (e.g., promoters and enhancers) and regulate transcription of an encoding DNA region. Thus, transcription factors can be used to modulate the expression of SUR1. Typically, a transcription factor comprises a binding domain that binds to DNA (a DNA-binding domain) and a regulatory domain that controls transcription. Where a regulatory domain activates transcription, that regulatory domain is designated an activation domain. Where that regulatory domain inhibits transcription, that regulatory domain is designated a repression domain. More specifically, transcription factors such as Sp1, HIF1, and NFB can be used to modulate expression of SUR1.

A transcription factor may be targeted by a composition. The transcription factor may be one that is associated with a pathway in which SUR1 is involved. The transcription factor may be targeted with an antagonist, including siRNA to downregulate the transcription factor. Such antagonists can be identified by standard methods in the art, and the antagonist is employed for treatment and or prevention of an individual in need thereof. The antagonist may be employed in conjunction with an additional compound, such as a composition that modulates the NC_{Ca-ATP} channel of the invention. For example, the antagonist may be used in combination with an inhibitor of the channel of the invention. When employed in combination, the antagonist of a transcription factor of a SUR1-related pathway may be administered prior to, during, and/or subsequent to the additional compound.

An antisense molecule that binds to a translational or transcriptional start site, or splice junctions, are ideal inhibitors. Antisense, ribozyme, and double-stranded RNA molecules target a particular sequence to achieve a reduction or elimination of a particular polypeptide, such as SUR1. Thus, it is disclosed that antisense, ribozyme, and double-stranded RNA, and RNA interference molecules are constructed and used to modulate SUR1 expression.

Antisense methodology takes advantage of the fact that nucleic acids tend to pair with complementary sequences. By complementary, it is meant that polynucleotides are those which are capable of base-pairing according to the standard Watson-Crick complementarity rules. That is, the larger purines will base pair with the smaller pyrimidines to form combinations of guanine paired with cytosine (G:C) and adenine paired with either thymine (A:T) in the case of DNA, or adenine paired with uracil (A:U) in the case of RNA. Inclusion of less common bases, such as inosine, 5-methylcytosine, 6-methyladenine, hypoxanthine and others, in hybridizing sequences does not interfere with pairing.

Targeting double-stranded (ds) DNA with polynucleotides leads to triple-helix formation; targeting RNA will lead to double-helix formation. Antisense polynucleotides, when introduced into a target cell, specifically bind to their target polynucleotide and interfere with transcription, RNA processing, transport, translation and/or stability. Antisense RNA constructs, or DNA encoding such antisense RNAs, are employed to inhibit gene transcription or translation or both within a host cell, either in vitro or in vivo, such as within a host animal, including a human subject.

Antisense constructs are designed to bind to the promoter and other control regions, exons, introns or even exon-intron boundaries of a gene. It is disclosed that the most effective antisense constructs may include regions complementary to intron/exon splice junctions. Thus, antisense constructs with complementarity to regions within 50-200 bases of an intron-exon splice junction may be used. It has been observed that some exon sequences can be included in the construct without seriously affecting the target selectivity thereof. The amount of exonic material included will vary depending on the particular exon and intron sequences used. One can readily test whether too much exon DNA is included simply by testing the constructs in vitro to determine whether normal cellular function is affected or whether the expression of related genes having complementary sequences is affected.

It is advantageous to combine portions of genomic DNA with cDNA or synthetic sequences to generate specific constructs. For example, where an intron is desired in the ultimate construct, a genomic clone will need to be used. The cDNA or a synthesized polynucleotide may provide more convenient restriction sites for the remaining portion of the construct and, therefore, would be used for the rest of the sequence.

It is also disclosed that double-stranded RNA is used as an interference molecule, e.g., RNA interference (RNAi). RNA interference is used to "knock down" or inhibit a particular gene of interest by simply injecting, bathing or feeding to the organism of interest the double-stranded RNA molecule. This technique selectively "knock downs" gene function without requiring transfection or recombinant techniques.

Another type of RNAi is often referred to as small interfering RNA (siRNA), which may also be utilized to inhibit SUR1. A siRNA may comprise a double stranded structure or a single stranded structure, the sequence of which is "substantially identical" to at least a portion of the target gene (see WO 04/046320). "Identity," as known in the art, is the relationship between two or more polynucleotide (or polypeptide) sequences, as determined by comparing the sequences. In the art, identity also means the degree of sequence relatedness between polynucleotide sequences, as determined by the match of the order of nucleotides between such sequences. Identity can be readily calculated. See, for example: Computational Molecular Biology, Lesk, A. M., ed. Oxford University Press, New York, 1988; Biocomputing: Informatics and Genome Projects, Smith, D. W., ea., Academic Press, New York, 1993, and the methods disclosed in WO 99/32619, WO 01/68836, WO 00/44914, and WO 01/36646. While a number of methods exist for measuring identity between two nucleotide sequences, the term is well known in the art. Methods for determining identity are typically designed to produce the greatest degree of matching of nucleotide sequence and are also typically embodied in computer programs. Such programs are readily available to those in the relevant art. For example, the GCG program package (Devereux et al.), BLASTP, BLASTN, and FASTA and CLUSTAL.

Thus, siRNA contains a nucleotide sequence that is essentially identical to at least a portion of the target gene, for example, SUR1, or any other molecular entity associated with the NC_{Ca-ATP} channel such as the pore-forming subunit. One of skill in the art is aware that the nucleic acid sequences for SUR1 are readily available in GenBank, for example, GenBank accession L40624. Preferably, the siRNA contains a nucleotide sequence that is completely identical to at least a portion of the target gene. Of course, when comparing an RNA sequence to a DNA sequence, an "identical" RNA sequence will contain ribonucleotides where the DNA sequence contains deoxyribonucleotides, and further that the RNA sequence will typically contain a uracil at positions where the DNA sequence contains thymidine.

One of skill in the art will appreciate that two polynucleotides of different lengths may be compared over the entire length of the longer fragment. Alternatively, small regions may be compared. Normally sequences of the same length are compared for a final estimation of their utility in the practice of the present invention. There may be 100% sequence identity between the dsRNA for use as siRNA and at least 15 contiguous nucleotides of the target gene (e.g., SUR1), although a dsRNA having 70%, 75%, 80%, 85%, 90%, or 95% or greater may also be used in the present invention. A siRNA that is essentially identical to a least a portion of the target gene may also be a dsRNA wherein one of the two complementary strands (or, in the case of a self-complementary RNA, one of the two self-complementary portions) is either identical to the sequence of that portion or the target gene or contains one or more insertions, deletions or single point mutations relative to the nucleotide sequence of that portion of the target gene. siRNA technology thus has the property of being able to tolerate sequence variations that might be expected to result from genetic mutation, strain polymorphism, or evolutionary divergence.

There are several methods for preparing siRNA, such as chemical synthesis, in vitro transcription, siRNA expression vectors, and PCR expression cassettes. Irrespective of which method one uses, the first step in designing an siRNA molecule is to choose the siRNA target site, which can be any site in the target gene. In certain embodiments, one of skill in the art may manually select the target selecting region of the gene, which may be an ORF (open reading frame) as the target selecting region and may preferably be 50-100 nucleotides downstream of the "ATG" start codon. However, there are several readily available programs available to assist with the design of siRNA molecules, for example siRNA Target Designer by Promega, siRNA Target Finder by GenScript Corp., siRNA Retriever Program by Imgenex Corp., EMBOSS siRNA algorithm, siRNA program by Qiagen, Ambion siRNA predictor, Ambion siRNA predictor, Whitehead siRNA prediction, and Sfold. Thus, it is envisioned that any of the above programs may be utilized to produce siRNA molecules.

Ribozymes are RNA-protein complexes that cleave nucleic acids in a site-specific fashion. Ribozymes have specific catalytic domains that possess endonuclease activity. For example, a large number of ribozymes accelerate phosphoester transfer reactions with a high degree of specificity, often cleaving only one of several phosphoesters in an oligonucleotide substrate. This specificity has been attributed to the requirement that the substrate bind via specific base-pairing interactions to the internal guide sequence ("IGS") of the ribozyme prior to chemical reaction. Ribozyme catalysis has primarily been observed as part of sequence specific cleavage/ligation reactions involving nucleic acids. For example, U.S. Pat. No. 5,354,855 reports that certain ribozymes can act as endonucleases with a sequence specificity greater than that of known ribonucleases and approaching that of the DNA restriction enzymes. Thus, sequence-specific ribozyme-mediated inhibition of gene expression is particularly suited to the therapeutic applications. Most of this work involved the modification of a target mRNA, based on a specific mutant codon that is cleaved by a specific ribozyme. In light of the information included herein and the knowledge of one of ordinary skill in the art, the preparation and use of additional ribozymes that are specifically targeted to a given gene will now be straightforward.

Designing and testing ribozymes for efficient cleavage of a target RNA is a process well known to those skilled in the art. The identification of operative and preferred sequences for use in SUR1 targeted ribozymes is simply a matter of preparing and testing a given sequence, and is a routinely practiced screening method known to those of skill in the art.

Following expression of individual regulatory and pore-forming subunit proteins of the channel, and in particular aspects of the invention, these proteins are modified by glycosylation in the Golgi apparatus of the cell, assembled into functional heteromultimers that comprise the channel, and then transported to the plasmalemmal membrane where they are inserted to form functional channels. The last of these processes is referred to as "trafficking."

In specific embodiments of the invention, molecules that bind to any of the constituent proteins interfere with post-translational assembly and trafficking, and thereby interfere with expression of functional channels. One such example is with glibenclamide binding to SUR1 subunits. In additional embodiments, glibenclamide, which binds with femtomolar affinity to SUR1, interferes with post-translational assembly and trafficking required for functional channel expression.

Disclosed are therapeutic methods employing compositions comprising the active substances disclosed herein. Preferably, these compositions include pharmaceutical compositions comprising a therapeutically effective amount of one or more of the active compounds or substances along with a pharmaceutically acceptable carrier.

As used herein, the term "pharmaceutically acceptable" carrier means a non-toxic, inert solid, semi-solid liquid filler, diluent, encapsulating material, formulation auxiliary of any type, or simply a sterile aqueous medium, such as saline. Some examples of the materials that can serve as pharmaceutically acceptable carriers are sugars, such as lactose, glucose and sucrose, starches such as corn starch and potato starch, cellulose and its derivatives such as sodium carboxymethyl cellulose, ethyl cellulose and cellulose acetate; powdered tragacanth; malt, gelatin, talc; excipients such as cocoa butter and suppository waxes; oils such as peanut oil, cottonseed oil, safflower oil, sesame oil, olive oil, corn oil and soybean oil; glycols, such as propylene glycol, polyols such as glycerin, sorbitol, mannitol and polyethylene glycol; esters such as ethyl oleate and ethyl laurate, agar; buffering agents such as magnesium hydroxide and aluminum hydroxide; alginic acid; pyrogen-free water; isotonic saline, Ringer's solution; ethyl alcohol and phosphate buffer solutions, as well as other non-toxic compatible substances used in pharmaceutical formulations.

Wetting agents, emulsifiers and lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. Examples of pharmaceutically acceptable antioxidants include, but are not limited to, water soluble antioxidants such as ascorbic acid, cysteine hydrochloride, sodium bisulfite, sodium metabisulfite, sodium sulfite, and the like; oil soluble antioxidants, such as ascorbyl palmitate, butylated hydroxyanisole (BHA), butylated hydroxytoluene (BHT), lecithin, propyl gallate, aloha-tocopherol and the like; and the metal chelating agents such as citric acid, ethylenediamine tetraacetic acid (EDTA), sorbitol, tartaric acid, phosphoric acid and the like.

The total daily dose of the active compounds administered to a subject in single or in divided doses can be in amounts, for example, from 0.01 to 25 mg/kg body weight or more usually from 0.1 to 15 mg/kg body weight. Single dose compositions may contain such amounts or submultiples thereof to make up the daily dose. In general, treatment regimens comprise administration to a human or other mammal in need of such treatment from about 1 mg to about 1000 mg of the active substance(s) of this invention per day in multiple doses or in a single dose of from 1 mg, 5 mg, 10 mg, 100 mg, 500 mg or 1000 mg.

For example, directed to a method of treating pain in a subject by administering to the subject a formulation containing an effective amount of a compound that blocks the NC_{Ca-ATP} channel and a pharmaceutically acceptable carrier. Such formulations may contain from about 0.1 to about 100 grams of tolbutamide or from about 0.5 to about 500 milligrams of glibenclamide.

The active agents of the present invention can be administered alone or in combination with one or more active pharmaceutical agents. In some embodiments, the one or more active pharmaceutical agents are useful to treat neuropathic pain in the subject.

Liquid dosage forms for oral administration may include pharmaceutically acceptable emulsions, microemulsions, solutions, suspensions, syrups and elixirs containing inert diluents commonly used in the art, such as water, isotonic solutions, or saline. Such compositions may also comprise adjuvants, such as wetting agents; emulsifying and suspending agents; sweetening, flavoring and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulation can be sterilized, for example, by filtration through a bacteria-retaining filter, or by incorporating sterilizing agents in the form of sterile solid compositions, which can be dissolved or dispersed in sterile water or other sterile injectable medium just prior to use.

In order to prolong the effect of a drug, it is often desirable to slow the absorption of a drug from subcutaneous or intramuscular injection. The most common way to accomplish this is to inject a suspension of crystalline or amorphous material with poor water solubility. The rate of absorption of the drug becomes dependent on the rate of dissolution of the drug, which is, in turn, dependent on the physical state of the drug, for example, the crystal size and the crystalline form. Another approach to delaying absorption of a drug is to administer the drug as a solution or suspension in oil. Injectable depot forms can also be made by forming microcapsule matrices of drugs and biodegradable polymers, such as polylactide-polyglycoside. Depending on the ratio of drug to polymer and the composition of the polymer, the rate of drug release can be controlled. Examples of other biodegradable polymers include polyorthoesters and polyanhydrides. The depot injectables can also be made by entrapping the drug in liposomes or microemulsions, which are compatible with body tissues.

Suppositories for rectal administration of the drug can be prepared by mixing the drug with a suitable non-irritating excipient, such as cocoa butter and polyethylene glycol which are solid at ordinary temperature but liquid at the rectal temperature and will, therefore, melt in the rectum and release the drug.

Solid dosage forms for oral administration may include capsules, tablets, pills, powders, gelcaps and granules. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such as magnesium stearate and microcrystalline cellulose. In the case of capsules, tablets and pills, the dosage forms may also comprise buffering agents. Tablets and pills can additionally be prepared with enteric coatings and other release-controlling coatings.

Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferably, in a certain part of the intestinal tract, optionally in a delayed manner. Examples of embedding compositions which can be used include polymeric substances and waxes.

Dosage forms for topical or transdermal administration of a compound of this invention further include ointments, pastes, creams, lotions, gels, powders, solutions, sprays, inhalants or patches. Transdermal patches have the added advantage of providing controlled delivery of active compound to the body. Such dosage forms can be made by dissolving or dispersing the compound in the proper medium. Absorption enhancers can also be used to increase the flux of the compound across the skin. The rate can be controlled by either providing a rate controlling membrane or by dispersing the compound in a polymer matrix or gel. The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

The therapeutic compound may be delivered transdermally. The term "transdermal delivery" as used herein means administration of the pharmaceutical composition topically to the skin wherein the active ingredient or its pharmaceutically acceptable salts, will be percutaneously delivered in a therapeutically effective amount.

The composition to be applied transdermally may further comprise an absorption enhancer. The term " absorption enhancer" as used herein means a compound which enhance the percutaneous absorption of drugs. These substances are sometimes also referred to as skin-penetration enhancers, accelerants, adjuvants and sorption promoters. Various absorption enhancers are known to be useful in transdermal drug delivery. U.S. Pat. Nos. 5,230,897, 4,863,970, 4,722,941, and 4,931,283 disclose some representative absorption enhancers used in transdermal compositions and for topical administration. In some embodiments, the absorption enhancer is N-lauroyl sarcosine, sodium octyl sulfate, methyl laurate, isopropyl myristate, oleic acid, glyceryl oleate or sodium lauryl sulfoacetate, or a combination thereof. The composition contains on a weight/volume (w/v) basis the absorption enhancer in an amount of about 1-20%, 1-15%, 1-10% or 1-5%. To enhance further the ability of the therapeutic agent(s) to penetrate the skin or mucosa, the composition can also contain a surfactant, an azone-like compound, an alcohol, a fatty acid or ester, or an aliphatic thiol.

The therapeutic compound may be delivered via a transdermal patch.
A transdermal patch may comprise an effective amount of the therapeutic compound for treating pain, e.g., glibenclamide. The transdermal patch may further comprise an absorption enhancer.

The transdermal composition can further comprise one or more additional excipients. Suitable excipients include without limitation solubilizers (e.g., C₂-C₈ alcohols), moisturizers or humectants (e.g., glycerol [glycerin], propylene glycol, amino acids and derivatives thereof, polyamino acids and derivatives thereof, and pyrrolidone carboxylic acids and salts and derivatives thereof), surfactants (e.g., sodium laureth sulfate and sorbitan monolaurate), emulsifiers (e.g., cetyl alcohol and stearyl alcohol), thickeners (e.g., methyl cellulose, ethyl cellulose, hydroxymethyl cellulose, hydroxypropyl cellulose, polyvinylpyrrolidone, polyvinyl alcohol and acrylic polymers), and formulation bases or carriers (e.g., polyethylene glycol as an ointment base). As a non-limiting example, the base or carrier of the composition can contain ethanol, propylene glycol and polyethylene glycol (e.g., PEG 300), and optionally an aqueous liquid (e.g., isotonic phosphate-buffered saline).

Compositions comprising at least one SUR1 antagonist compound (for use as defined in the claims) and a pharmaceutically acceptable carrier may be contemplated.

Exemplary pharmaceutically acceptable carriers include carriers suitable for oral, intravenous, subcutaneous, intramuscular, intracutaneous, and the like administration. Administration in the form of creams, lotions, tablets, dispersible powders, granules, syrups, elixirs, sterile aqueous or non-aqueous solutions, suspensions or emulsions, and the like, is contemplated.

For the preparation of oral liquids, suitable carriers include emulsions, solutions, suspensions, syrups, and the like, optionally containing additives such as wetting agents, emulsifying and suspending agents, sweetening, flavoring and perfuming agents, and the like.

For the preparation of fluids for parenteral administration, suitable carriers include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized, for example, by filtration through a bacteria-retaining filter, by incorporating sterilizing agents into the compositions, by irradiating the compositions, or by heating the compositions. They can also be manufactured in the form of sterile water, or some other sterile injectable medium immediately before use. The active compound is admixed under sterile conditions with a pharmaceutically acceptable carrier and any needed preservatives or buffers as may be required.

The treatments may include various "unit doses." Unit dose is defined as containing a predetermined quantity of the therapeutic composition (an antagonist of the NC_{Ca-ATP} channel or its related-compounds thereof) calculated to produce the desired responses in association with its administration, e.g., the appropriate route and treatment regimen. The quantity to be administered, and the particular route and formulation, are within the skill of those in the clinical arts. Also of importance is the subject to be treated, in particular, the state of the subject and the protection desired. A unit dose need not be administered as a single injection but may comprise continuous infusion over a set period of time.

Pharmaceutical compositions of the present invention may comprise an effective amount of one or more modulators of NC_{Ca-ATP} channel (or related compounds or additional agent) dissolved or dispersed in a pharmaceutically acceptable carrier. The phrases "pharmaceutical or pharmacologically acceptable" refers to molecular entities and compositions that do not produce an adverse, allergic or other untoward reaction when administered to an animal, such as, for example, a human, as appropriate. The preparation of a pharmaceutical composition that contains at least one modulators of NC.sub.Ca-ATP channel (antagonist and/or agonist) or related compounds or additional active ingredient will be known to those of skill in the art in light of the present disclosure, as exemplified by Remington's Pharmaceutical Sciences, 18th Ed. Mack Printing Company, 1990. Moreover, for animal (e.g., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the pharmaceutical compositions is contemplated.

The inhibitors of the NC_{Ca-ATP} channel may comprise different types of carriers depending on whether it is to be administered in solid, liquid or aerosol form, and whether it need to be sterile for such routes of administration as injection. The present invention can be administered intravenously, intradermally, transdermally, intrathecally, intraventricularly, intraarterially, intraperitoneally, intranasally, intravaginally, intrarectally, topically, intramuscularly, subcutaneously, mucosally, orally, topically, locally, inhalation (e.g., aerosol inhalation), injection, infusion, continuous infusion, localized perfusion bathing target cells directly, via a catheter, via a lavage, in cremes, in lipid compositions (e.g., liposomes), or by other method or any combination of the forgoing as would be known to one of ordinary skill in the art (see, for example, Remington's Pharmaceutical Sciences, 18th Ed. Mack Printing Company, 1990).

The inhibitors of the NC_{Ca-ATP} channel may be formulated into a composition in a free base, neutral or salt form. Pharmaceutically acceptable salts, include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Upon formulation, solutions will be administered in a manner compatible with the dosage formulation and in such amount as is therapeutically effective. The formulations are easily administered in a variety of dosage forms such as formulated for parenteral administrations such as injectable solutions, or aerosols for delivery to the lungs, or formulated for alimentary administrations such as drug release capsules and the like.

Further in accordance with the present invention, the composition of the present invention suitable for administration is provided in a pharmaceutically acceptable carrier with or without an inert diluent. The carrier should be assimilable and includes liquid, semi-solid, i.e., pastes, or solid carriers. Except insofar as any conventional media, agent, diluent or carrier is detrimental to the recipient or to the therapeutic effectiveness of the composition contained therein, its use in administrable composition for use in practicing the methods of the present invention is appropriate. Examples of carriers or diluents include fats, oils, water, saline solutions, lipids, liposomes, resins, binders, fillers and the like, or combinations thereof. The composition may also comprise various antioxidants to retard oxidation of one or more component. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof.

In accordance with the present invention, the composition is combined with the carrier in any convenient and practical manner, i.e., by solution, suspension, emulsification, admixture, encapsulation, absorption and the like. Such procedures are routine for those skilled in the art.

The composition my be combined or mixed thoroughly with a semi-solid or solid carrier. The mixing can be carried out in any convenient manner such as grinding. Stabilizing agents can be also added in the mixing process in order to protect the composition from loss of therapeutic activity, i.e., denaturation in the stomach. Examples of stabilizers for use in the composition include buffers, amino acids such as glycine and lysine, carbohydrates such as dextrose, mannose, galactose, fructose, lactose, sucrose, maltose, sorbitol, mannitol, etc.

The present invention may concern the use of a pharmaceutical lipid vehicle compositions that include inhibitors of the NC_{Ca-ATP} channel, one or more lipids, and an aqueous solvent. As used herein, the term "lipid" will be defined to include any of a broad range of substances that is characteristically insoluble in water and extractable with an organic solvent. This broad class of compounds are well known to those of skill in the art, and as the term "lipid" is used herein, it is not limited to any particular structure. Examples include compounds which contain long-chain aliphatic hydrocarbons and their derivatives. A lipid may be naturally occurring or synthetic (i.e., designed or produced by man). However, a lipid is usually a biological substance. Biological lipids are well known in the art, and include for example, neutral fats, phospholipids, phosphoglycerides, steroids, terpenes, lysolipids, glycosphingolipids, glycolipids, sulphatides, lipids with ether and ester-linked fatty acids and polymerizable lipids, and combinations thereof. Of course, compounds other than those specifically described herein that are understood by one of skill in the art as lipids are also encompassed by the compositions and methods of the present invention.

One of ordinary skill in the art would be familiar with the range of techniques that can be employed for dispersing a composition in a lipid vehicle. For example, the inhibitors of the NC_{Ca-ATP} channel may be dispersed in a solution containing a lipid, dissolved with a lipid, emulsified with a lipid, mixed with a lipid, combined with a lipid, covalently bonded to a lipid, contained as a suspension in a lipid, contained or complexed with a micelle or liposome, or otherwise associated with a lipid or lipid structure by any means known to those of ordinary skill in the art. The dispersion may or may not result in the formation of liposomes.

The actual dosage amount of a composition of the present invention administered to an animal patient can be determined by physical and physiological factors such as body weight, severity of condition, the type of disease being treated, previous or concurrent therapeutic and/or prophylactic interventions, idiopathy of the patient and on the route of administration. Depending upon the dosage and the route of administration, the number of administrations of a preferred dosage and/or an effective amount may vary according to the response of the subject. The practitioner responsible for administration will, in any event, determine the concentration of active ingredient(s) in a composition and appropriate dose(s) for the individual subject.

Pharmaceutical compositions may comprise, for example, at least about 0.1% of an active compound. An active compound may comprise between about 2% to about 75% of the weight of the unit, or between about 25% to about 60%, for example, and any range derivable therein. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

In some embodiments of the present invention, the inhibitors of the NC_{Ca-ATP} channel are formulated to be administered via an alimentary route. Alimentary routes include all possible routes of administration in which the composition is in direct contact with the alimentary tract. Specifically, the pharmaceutical compositions disclosed herein may be administered orally, buccally, rectally, or sublingually. As such, these compositions may be formulated with an inert diluent or with an assimilable edible carrier or they may be enclosed in hard- or soft-shell gelatin capsule, or they may be compressed into tablets, or they may be incorporated directly with the food of the diet.

In certain embodiments, the active compounds may be incorporated with excipients and used in the form of ingestible tablets, buccal tables, troches, capsules, elixirs, suspensions, syrups, wafers, and the like U.S. Pat. Nos. 5,641,515; 5,580,579 and 5,792,451). The tablets, troches, pills, capsules and the like may also contain the following: a binder, such as, for example, gum tragacanth, acacia, cornstarch, gelatin or combinations thereof; an excipient, such as, for example, dicalcium phosphate, mannitol, lactose, starch, magnesium stearate, sodium saccharine, cellulose, magnesium carbonate or combinations thereof; a disintegrating agent, such as, for example, corn starch, potato starch, alginic acid or combinations thereof; a lubricant, such as, for example, magnesium stearate; a sweetening agent, such as, for example, sucrose, lactose, saccharin or combinations thereof; a flavoring agent, such as, for example peppermint, oil of wintergreen, cherry flavoring, orange flavoring, etc. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. When the dosage form is a capsule, it may contain, in addition to materials of the above type, carriers such as a liquid carrier. Gelatin capsules, tablets, or pills may be enterically coated. Enteric coatings prevent denaturation of the composition in the stomach or upper bowel where the pH is acidic. See, e.g., U.S. Pat. No. 5,629,001. Upon reaching the small intestines, the basic pH therein dissolves the coating and permits the composition to be released and absorbed by specialized cells, e.g., epithelial enterocytes and Peyer's patch M cells. A syrup of elixir may contain the active compound sucrose as a sweetening agent methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations.

For oral administration the compositions of the present invention may alternatively be incorporated with one or more excipients in the form of a mouthwash, dentifrice, buccal tablet, oral spray, or sublingual orally-administered formulation. For example, a mouthwash may be prepared incorporating the active ingredient in the required amount in an appropriate solvent, such as a sodium borate solution (Dobell's Solution). Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively, the compositions may be fashioned into a tablet or solution form that may be placed under the tongue or otherwise dissolved in the mouth.

Additional formulations that are suitable for other modes of alimentary administration include suppositories. Suppositories are solid dosage forms of various weights and shapes, usually medicated, for insertion into the rectum. After insertion, suppositories soften, melt or dissolve in the cavity fluids. In general, for suppositories, traditional carriers may include, for example, polyalkylene glycols, triglycerides or combinations thereof. In certain embodiments, suppositories may be formed from mixtures containing, for example, the active ingredient in the range of about 0.5% to about 10%, and preferably about 1% to about 2%.

In further embodiments, the inhibitors of the NC_{Ca-ATP} channel may be administered via a parenteral route. As used herein, the term "parenteral" includes routes that bypass the alimentary tract. Specifically, the pharmaceutical compositions disclosed herein may be administered for example, but not limited to intravenously, intradermally, transdermally, intramuscularly, intraarterially, intraventricularly, intrathecally, subcutaneous, or intraperitoneally U.S. Pat. Nos. 6,7537,514; 6,613,308; 5,466,468; 5,543,158; 5,641,515; and 5,399,363.

The therapeutic compound may be administered intrathecally. The compound may be administered intrathecally via an implantable pump. The implantable pump comprises a SynchroMed^{™} II pump that stores and delivers medication into the intrathecal space (Medtronic).

Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable use include sterile aqueous solutions or dispersions and sterile powders for the extemporaneous preparation of sterile injectable solutions or dispersions (U.S. Pat. No. 5,466,468). In all cases the form must be sterile and must be fluid to the extent that easy injectability exists. It must be stable under the conditions of manufacture and storage and must be preserved against the contaminating action of microorganisms, such as bacteria and fungi. The carrier can be a solvent or dispersion medium containing, for example, water, ethanol, DMSO, polyol (i.e., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous, and intraperitoneal administration. In this connection, sterile aqueous media that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. A powdered composition is combined with a liquid carrier such as, e.g., water or a saline solution, with or without a stabilizing agent.

In other embodiments, the inhibitors of the NC_{Ca-ATP} channel may be formulated for administration via various miscellaneous routes, for example, topical (i.e., transdermal) administration, mucosal administration (intranasal, vaginal, etc.) and/or inhalation.

Pharmaceutical compositions for topical administration may include the active compound formulated for a medicated application such as an ointment, paste, cream or powder. Ointments include all oleaginous, adsorption, emulsion and water-solubly based compositions for topical application, while creams and lotions are those compositions that include an emulsion base only. Topically administered medications may contain a penetration enhancer to facilitate adsorption of the active ingredients through the skin. Suitable penetration enhancers include glycerin, alcohols, alkyl methyl sulfoxides, pyrrolidones and luarocapram. Possible bases for compositions for topical application include polyethylene glycol, lanolin, cold cream and petrolatum as well as any other suitable absorption, emulsion or water-soluble ointment base. Topical preparations may also include emulsifiers, gelling agents, and antimicrobial preservatives as necessary to preserve the active ingredient and provide for a homogenous mixture. Transdermal administration of the present invention may also comprise the use of a "patch". For example, the patch may supply one or more active substances at a predetermined rate and in a continuous manner over a fixed period of time.

In certain embodiments, the pharmaceutical compositions may be delivered by eye drops, intranasal sprays, inhalation, and/or other aerosol delivery vehicles. Methods for delivering compositions directly to the lungs via nasal aerosol sprays has been described e.g., in U.S. Pat. Nos. 5,756,353 and 5,804,212. Likewise, the delivery of drugs using intranasal microparticle resins and lysophosphatidyl-glycerol compounds (U.S. Pat. No. 5,725,871) are also well-known in the pharmaceutical arts. Likewise, transmucosal drug delivery in the form of a polytetrafluoroetheylene support matrix is described in U.S. Pat. No. 5,780,045.

The term aerosol refers to a colloidal system of finely divided solid of liquid particles dispersed in a liquefied or pressurized gas propellant. The typical aerosol of the present invention for inhalation will consist of a suspension of active ingredients in liquid propellant or a mixture of liquid propellant and a suitable solvent. Suitable propellants include hydrocarbons and hydrocarbon ethers. Suitable containers will vary according to the pressure requirements of the propellant. Administration of the aerosol will vary according to subject's age, weight and the severity and response of the symptoms.

### EXAMPLES

### EXAMPLE 1 - Sur1/Glibenclamide in Neuropathic Pain.

WT mice underwent unilateral PNI by sciatic nerve cuffing, a widely accepted model of neuropathic pain in mice (Valcin et al., The sciatic nerve cuffing model of neuropathic pain in mice, J Vis Exp, PMID: 25078668 (2014)). Sciatic nerve cuffing reliably induces signs of chronic pain, including sustained mechanical and thermal allodynia. In this model, mechanical allodynia assessed using von Frey filaments develops within 1 week, and is sustained thereafter.

Dorsal horn astrocytes upregulate Sur1-Trpm4, IL-6, CCL2 and CXCL1. Immunohistochemistry showed upregulation of Sur1 and Trpm4 in dorsal horn astrocytes, which was absent in contralateral astrocytes (Fig. 1A-C, E). FRET showed that the two subunits co-assembled to form heterodimers required for functional channels (Fig. 1D). qPCR confirmed upregulation of *Abcc8* and *Trpm4* mRNA (Fig. 1E).

Immunohistochemistry also showed prominent upregulation of IL-6, CCL2 and CXCL1 in ipsilateral dorsal horn astrocytes, which was absent in contralateral astrocytes (Fig. 2A- D), consistent with previous reports.

***Abcc8*^{loxP/loxP}/Gfap-Cre mice with astrocyte-specific deletion of *Abcc8,* the gene that encodes Sur1, exhibit significant protection from PNI-induced allodynia.** To examine the possible function of Sur1 in astrocytes, we subjected *Abcc8*^{loxP/loxP}/Gfap-Cre mice to PNI, with littermate WT-Cre mice as controls. In controls, mechanical allodynia was well established by 1 week (Fig. 3A). Remarkably, in *Abcc8*^{loxP/loxP}/Gfap-Cre mice, allodynia failed to develop during 2 weeks after PNI (Fig. 3A). However, performance on the accelerating rotarod was equally impaired in *Abcc8*^{loxP/loxP}/Gfap-Cre and controls, consistent with equivalent nerve injury. These data, together with the immunohistochemistry data (Fig. 1), strongly implicate Sur1-Trpm4 channels in astrocytes in the development of allodynia post-PNI.

**Inhibiting Sur1-Trpm4 with (non-addicting) glibenclamide reduces PNI-induced allodynia and IL-6, CCL2 and CXCL1 expression by dorsal horn astrocytes.** The salutary effects of genetic deletion of *Abcc8* suggested that pharmacological inhibition of Sur1 by glibenclamide may be beneficial. Although the normal blood-brain barrier is poorly permeable to glibenclamide, (Lahmann et al., Systemic Administration of Glibenclamide Fails to Achieve Therapeutic Levels in the Brain and Cerebrospinal Fluid of Rodents, PLoS One, 10:e0134476. PMID: 26225433 (2015). in CNS inflammatory conditions such as experimental autoimmune encephalomyelitis, glibenclamide readily enters spinal cord tissues where it ameliorates inflammation (Schattling et al., TRPM4 cation channel mediates axonal and neuronal degeneration in experimental autoimmune encephalomyelitis and multiple sclerosis, Nat Med, 18:1805-11, PMID: 23160238 (2012); Makar et al., Silencing of Abcc8 or inhibition of newly upregulated Sur1- Trpm4 reduce inflammation and disease progression in experimental autoimmune encephalomyelitis, J Neuroinflammation, 12:210, PMID: 26581714 (2015)).

Administering glibenclamide (10 µg IP loading dose plus 40 ng/hr subcutaneous via mini-osmotic pump x 2 weeks) shortly after PNI resulted in significant blunting of mechanical and cold allodynia (Fig. 3B). Moreover, administering glibenclamide beginning on day 18 after PNI, after allodynia was well established, resulted in significant reversal of symptoms (Fig. 3C).

Quantitative immunohistochemistry in these mice showed significant reduction of IL-6, CCL2 and CXCL1 in ipsilateral dorsal horn astrocytes, compared to vehicle-treated controls (Fig. 2D).

Importantly, unlike opioids, glibenclamide does not modify normal mechanical or thermal sensitivity thresholds (not shown), but exerts antihypersensitivity effects (antiallodynic) only in a sensitizing condition such as PNI.

### Methods

***Sciatic nerve cuff model of neuropathic pain*** (Valcin et al., The sciatic nerve cuffing model of neuropathic pain in mice, J Vis Exp, PMID: 25078668 (2014)). Surgeries are performed under ketamine/xylazine anesthesia using aseptic techniques. The common branch of the right sciatic nerve is dissected and a 2 mm long section of pre-split PE-20 tubing is placed around it. Shams undergo the same surgery without cuffing.

### Tests for chronic pain.

Mechanical hypersensitivity. The threshold for ipsi- and contralateral hindpaw withdrawal is determined using von Frey filaments (Chaplan et al., Quantitative assessment of tactile allodynia in the rat paw, J Neurosci Methods, 53:55-63, PMID: 7990513(1994)).Mice are placed in clear Plexiglas boxes on an elevated mesh screen. Calibrated von Frey filaments are applied to the plantar surface of each hindpaw (0.16-15 gm). The "up-down method" (Chaplan et al., Quantitative assessment of tactile allodynia in the rat paw, J Neurosci Methods, 53:55-63, PMID: 7990513(1994)) is used to determine the withdrawal threshold.

Cold allodynia is assessed using acetone. A 10 µL volume of acetone is propelled via air burst from a 200 µL pipette onto the mid-plantar surface of the hind paw without touching the skin. Time spent elevating/licking/biting/shaking the stimulated paw, up to 15 sec, is measured with a stop watch. Each paw is stimulated 5x, allowing 5 min to elapse between successive paw stimulations.

Tissue analysis. Spinal cords are perfusion-fixed (4% PFA), cryoprotected and cryosectioned coronally (7 µm). The dorsal horns of L3-4, which innervates the plantar surface of the mouse hindpaw (Rigaud et al., Species and strain differences in rodent sciatic nerve anatomy: implications for studies of neuropathic pain, Pain, 136:188-201, PMID: 18316160 (2008)), are examined for markers of astrocyte activation (GFAP), Sur1-Trpm4 and IL-6, as well as the chemokines CCL2 and CXCL1. Tissues are immunolabeled using commercial primary antibodies, as we described (Kurland et al., The Sur1-Trpm4 channel regulates NOS2 transcription in TLR4-activated microglia, J Neuroinflammation, 13:130, PMID: 27246103 (2016)). Immuno- FRET (Forster Resonance Energy Transfer). Immuno-FRET, which is used to determine heteromeric co-assembly of Sur1 and Trpm4, is performed as we described (Mehta et al., Sur1-Trpm4 Cation Channel Expression in Human Cerebral Infarcts, J Neuropathol Exp Neural, 74:835-49, PMID: 26172285 (2015)). Immunoblot and qPCR. Fresh tissues are flash-frozen and are processed for immunoblot and qPCR as we described (Kurland et al., The Sur1-Trpm4 channel regulates NOS2 transcription in TLR4-activated microglia, J Neuroinflammation, 13:130, PMID: 27246103 (2016)).

### EXAMPLE 2 - Glibenclamide reduces pain behaviors after PNI.

WT mice underwent sciatic n. cuffing (Yalcin et al., The sciatic nerve cuffing model of neuropathic pain in mice, J Vis Exp, PMID 25078668 (2014)). and on day21, were treated with either vehicle or glibenclamide. In controls, mechanical (von Frey filaments) and thermal (acetone) allodynia developed within 1 week of PNI (Fig. 10A,B, red vs. black). In these mice, anxiety (open field and elevated plus maze) and depression-like behavior (tail suspension) were significantly abnormal compared to naive uninjured mice (Fig. 10C-E).

In mice treated with glibenclamide (10 µg/day intraperitoneal [**IP**]) starting on day21, both mechanical and thermal allodynia, as well as anxiety and depression-like behaviors were significantly ameliorated by glibenclamide (Fig. 10C-E, green). Note the gradual but complete reversal of mechanical allodynia that required 2-3 weeks of drug treatment (Fig. 10A).

***Deletion of SUR1 and TRPM4 reduces pain behaviors after PNI.*** The salutary effects of glibenclamide noted above could be due to blockade of either K_{ATP} (SUR1-KIR6.2) or SUR1-TRPM4 channels or an off-target effect. We studied sciatic n. cuffing in *Abcc8* knock-out (*Abcc8*KO) and *Trpm4* knock-out (*Trpm4*KO) mice, with WT mice used as controls. Mechanical allodynia (von Frey filaments) developed within 1 week in WT controls, as above, but failed to develop in either *Abcc8*KO or *Trpm4*KO mice (Fig. 11). Thermal hyperalgesia (Hargreaves method) also developed within 1 week in WT mice, but failed to develop in either *Abcc8*KO or *Trpm4KO* mice (Fig. 11). These data strongly support the hypothesis that glibenclamide acts via SUR1-TRPM4, not via K_{ATP} or an off-target mechanism.

***SUR1-TRPM4 upregulation in dorsal horn astrocytes.*** Spinal cord tissues from untreated mice with PNI from the experiment of Fig. 10 were examined after euthanasia on day49 to determine expression of SUR1-TRPM4. Ipsilateral but not contralateral dorsal horn astrocytes showed marked upregulation of SUR1 and TRPM4 (Fig. 5A,B,D). Förster Resonance Energy Transfer (**FRET**) showed that the two ion channel subunits co-assembled to form heterodimers, consistent with the formation of functional SUR1-TRPM4 channels (Fig. 5E). Immunoblot and qPCR confirmed upregulation of channel subunits (Fig. 5F). By contrast, co-labeling for Iba1 showed minimal SUR1 in microglia/macrophages (Fig. 5C).

***Glibenclamide decreases IL-6, CCL2 and CXCL1 in dorsal horn astrocytes.*** Spinal cord tissues from mice from the experiment of Fig. 10 also were examined for cytokines/chemokines. In vehicle-treated mice, ipsilateral but not contralateral dorsal horn astrocytes showed prominent activation (GFAP upregulation), and upregulation of IL-6, CCL2 and CXCL1 (Fig. 6A-C), as previously reported (Zhou et al., Interleukin-6: an emerging regulator of pathological pain, J Neuroinflammation, 13:141, PMID 27267059 (2016); Menetski et al., Mice overexpressing chemokine ligand 2 (CCL2) in astrocytes display enhanced nociceptive responses, Neuroscience, 149:706-14, PMID 17870246 (2007); Zhang et al., Chemokine contribution to neuropathic pain: respective induction of CXCL1 and CXCR2 in spinal cord astrocytes and neurons, Pain, 154:2185-97, PMID 23831863 (2013)), and in keeping with the general notion of a neuroinflammatory response within the spinal cord following PNI (Gordh et al., Chronic spinal nerve ligation induces microvascular permeability disturbances, astrocytic reaction, and structural changes in the rat spinal cord, Acta Neurochir Suppl, 96:335-40, PMID 16671481 (2006); Echeverry et al., Peripheral nerve injury alters blood-spinal cord barrier functional and molecular integrity through a selective inflammatory pathway, J Neurosci, 31:10819-28, PMID 21795534 (2011)). Co-labeling for Iba1 showed that cytokine and chemokine expression was absent in microglia (not shown), perhaps due to the late times (7 weeks post-PNI) when the tissues were examined.

In mice from Fig. 10 that were treated with glibenclamide, analysis of spinal cord tissues showed that ipsilateral dorsal horn astrocytes exhibited significant reductions in IL-6, CCL2 and CXCL1 (Fig. 6D), consistent with glibenclamide reducing astrocyte expression of these cytokines/chemokines.

***Glibenclamide enters the inflamed dorsal horn.*** Normally the BBB is not permeable to glibenclamide (Lahmann et al., Systemic Administration of Glibenclamide Fails to Achieve Therapeutic Levels in the Brain and Cerebrospinal Fluid of Rodents, PLoS One, 10:e0134476, PMID 26225433 (2015)), but in PNI, the blood-spinal cord barrier is leaky, with extravasation of serum proteins that persists at least 4-8 weeks (Gordh et al., Chronic spinal nerve ligation induces microvascular permeability disturbances, astrocytic reaction, and structural changes in the rat spinal cord, Acta Neurochir Suppl, 96:335-40, PMID 16671481 (2006); Echeverry et al., Peripheral nerve injury alters blood-spinal cord barrier functional and molecular integrity through a selective inflammatory pathway, J Neurosci, 31:10819-28, PMID 21795534 (2011)). In accord with this, we found abundant IgG and fluorescent bodipy-glibenclamide within the dorsal horn 3 weeks post-PNI (Fig. 12).

***Dorsal root Ganglion (DRG).*** Apart from dorsal horn, we also studied the DRG, where neurons express K_{ATP}. After PNI, neurons showed minimal changes in SUR1. However, DRG satellite cells, which co-label for glutamine synthase, showed marked upregulation of SUR1 compared to contralateral DRG (Fig. 13A,B,C). Moreover, SUR1 upregulation in DRG satellite cells was accompanied by upregulation of TRPM4 (Fig. 13D).

***Deletion of SUR1 in all GFAP-expressing cells.*** SUR1 expression in glia was previously implicated in key pathogenic signaling ((Makar et al.,Silencing of Abcc8 or inhibition of newly upregulated Sur1-Trpm4 reduce inflammation and disease progression in experimental autoimmune encephalomyelitis, J Neuroinflammation, 12:210, PMID 26581714 (2015); Gerzanich et al., Salutary effects of glibenclamide during the chronic phase of murine experimental autoimmune encephalomyelitis, J Neuroinflammation, 14:177, PMID 28865458 (2017)). We developed the *Abcc8*^{loxP/loxP}/*GFAP*-Cre/ERT2 mouse described below to study PNI and neuropathic pain, and specifically to address the anatomical and cellular location of SUR1 that is being targeted by glibenclamide.

The *Abcc8*^{loxP/loxP}/*GFAP*-Cre/ERT2 mouse is based on Abcc8-flox mice (loxP sites inserted to flank exon 2 of the *Abcc8* gene) on a C57BL6 background, generated and characterized by Nakamura and Bryan (Nakamura et al., Targeting SUR1/Abcc8-type neuroendocrine KATP Channels in Pancreatic Islet Cells, PLoS One, 9:e91525, PMID 24621811 (2014)). They are crossed with the *GFAP* promoter-driven Cre/ERT2 mouse (B6.Cg-Tg(GFAP-cre/ERT2)505Fmv/J; stock number 012849; The Jackson Laboratory; Bar Harbor, ME). Nakamura and Bryan (Nakamura et al., Targeting SUR1/Abcc8-type neuroendocrine KATP Channels in Pancreatic Islet Cells, PLoS One, 9:e91525, PMID 24621811 (2014)), reported that insertion of loxP sites did not affect the general phenotype of *Abcc8-*flox animals (Nakamura et al., Targeting SUR1/Abcc8-type neuroendocrine KATP Channels in Pancreatic Islet Cells, PLoS One, 9:e91525, PMID 24621811 (2014)). Genotyping of all KO animals is performed by an experienced, highly reliable, commercial firm (Transnetyx, Cordova TN).

The loss of *Abcc8*/SUR1 in astrocytes does not result in any overt developmental defect or phenotype, since global KO in *Abcc8*^{*-*/*-*} mice, which we studied previously (Makar et al., Silencing of Abcc8 or inhibition of newly upregulated Sur1-Trpm4 reduce inflammation and disease progression in experimental autoimmune encephalomyelitis, J Neuroinflammation, 12:210, PMID 26581714 (2015); Simard et al., Brief suppression of Abcc8 prevents autodestruction of spinal cord after trauma, Sci Transl Med, 2:28ra29, PMID 20410530 (2010)), shows no CNS phenotype (Seghers et al., Sur1 knockout mice. A model for K(ATP) channel-independent regulation of insulin secretion, J Biol Chem, 275:9270-7, PMID 10734066 (2000)) in the absence of CNS injury. The Cre-lox GFAP strain has been well characterized, especially in the spinal cord, and has been used extensively for targeting Cre-mediated recombination in glia. In our experiments, Cre recombinase expression by itself did not appear to contribute to the phenotype of interest - there were no differences in astrocytic expression of *Abcc8*/SUR1 or CCL2 between WT and *GFAP*-Cre/ERT2 mice.

We validated the *Abcc8*^{loxP/loxP}/*GFAP*-Cre/ERT2 mice ourselves, showing that, after ERT2 activation, SUR1 expression is virtually absent in dorsal horn astrocytes 10-days post-injury, whereas WT mice exhibit robust SUR1 expression (Fig. 14).

Controls for GFAP-specific KO experiments are WT Cre/ERT2 siblings (*Abcc8*^{+/+}/*GFAP*-Cre/ERT2), to compensate for any unobserved but possible *GFAP-*Cre/ERT2 phenotype.

We performed experiments with *Abcc8*^{loxP/loxP}/GFAP-Cre/ERT2 mice, with tamoxifen treatment (75 mg/kg IP × 5 days) followed by sciatic n. cuffing; littermate Cre/ERT2 heterozygote and WT mice treated with tamoxifen served as controls. In controls, mechanical allodynia was well established by 1 week (Fig. 15A). In *Abcc8*^{loxP/loxP}/GFAP-Cre/ERT2 mice, mechanical allodynia failed to develop during 2 weeks after PNI (Fig. 15A). Performance on the accelerating rotorod was equally impaired in KOs and controls, and histological examination of the sciatic nerves showed equivalent nerve pathology (edema, degraded myelin and infiltrating mononuclear cells) (Zhang et al., Neuroprotective effect of liquiritin against neuropathic pain induced by chronic constriction injury of the sciatic nerve in mice, Biomed Pharmacother, 95:186-198, PMID 28843150 (2017)) (Fig. 15C,D), consistent with equivalent nerve injury at these early times, despite absence of allodynia.

***Deletion of SUR1 in GFAP-expressing cells of the dorsal horn vs. sciatic nerve.*** After PNI, GFAP is upregulated not only by dorsal horn astrocytes, but by peripheral glial cells, including dorsal root ganglion (**DRG**) satellite cells (Triolo et al., Loss of glial fibrillary acidic protein (GFAP) impairs Schwann cell proliferation and delays nerve regeneration after damage, J Cell Sci, 119:3981-93, PMID 16988027 (2006)) and non-myelinating Schwann cells (Triolo et al., Loss of glial fibrillary acidic protein (GFAP) impairs Schwann cell proliferation and delays nerve regeneration after damage, J Cell Sci, 119:3981-93, PMID 16988027 (2006)) in the sciatic n. Moreover, K_{ATP} channels (with SUR1 subunits) are expressed in the DRG (Zoga et al., KATP channel subunits in rat dorsal root ganglia: alterations by painful axotomy, Mol Pain, 6:6, PMID 20102598 (2010)) and in paranodal sites of nodes of Ranvier and the Schmidt-Lanterman incisures of Schwann cells. However, K_{ATP} channels mediate anti-nociception mediated by analgesics. Thus, it is unlikely that the salutary effects of SUR1 deletion could have been due to downregulation of K_{ATP} channels in GFAP-expressing cells in the DRG or sciatic n.

Nevertheless, to investigate potential involvement of SUR1 in peripheral vs. central glia, we studied cell-&-site-specific gene knockdown of SUR1 using *Abcc8*^{loxP/loxP}/*GFAP-*Cre/ERT2 mice with local ERT2 activation. Endoxifen (Felker et al., In Vivo Performance and Properties of Tamoxifen Metabolites for CreERT2 Control, PLoS One, 11:e0152989, PMID 27077909 (2016)), an active metabolite of tamoxifen, was administered either directly on the sciatic n. using a drug-eluting perineural device (Zadelaar et al., Local Cre-mediated gene recombination in vascular smooth muscle cells in mice, Transgenic Res, 15:31-6, PMID 16475008 (2006)), or was administered intrathecally at L6-S1 using a brain infusion kit. Sciatic n. cuffing followed on day7. Our experiments showed that, with nerve application, SUR1 was downregulated (Fig. 16A-D), but this did not protect from mechanical allodynia (Fig. 16E). By contrast, intrathecal delivery of endoxifen effectively prevented mechanical allodynia (Fig. 16E). Importantly, intrathecal infusion in naive mice does not induce inflammation (Fig. 16F).

Together, these experiments suggest that glibenclamide is highly promising for the treatment of certain manifestations of neuropathic pain following PNI, and that the critical site of action of glibenclamide appears to be at dorsal horn astrocytes. The experiments of this Example are designed to confirm and expand upon these data.

Glibenclamide normally is excluded from the uninjured CNS (Lahmann et al., Systemic Administration of Glibenclamide Fails to Achieve Therapeutic Levels in the Brain and Cerebrospinal Fluid of Rodents, PLoS One, 10:e0134476, PMID 26225433 (2015)). However, in data developed for this example, we show that glibenclamide does in fact enter into the spinal cord at the segments from which the injured nerve originates (Fig. 12), which is in agreement with reports that neuroinflammation within the spinal cord induced by PNI is characterized by BBB leakage (Gordh et al., Chronic spinal nerve ligation induces microvascular permeability disturbances, astrocytic reaction, and structural changes in the rat spinal cord, Acta Neurochir Suppl, 96:335-40, PMID 16671481 (2006); Echeverry et al., Peripheral nerve injury alters blood-spinal cord barrier functional and molecular integrity through a selective inflammatory pathway, J Neurosci, 31:10819-28, PMID 21795534 (2011)).

In oral pill form, glibenclamide is not appropriate for use by non-diabetic patients, due to the risk of hypoglycemia. However, infusion of an appropriate dose of injectable glibenclamide has been shown to be safe in non-diabetics, yielding serum levels below the hypoglycemic threshold (~45 ng/mL) (Sheth et al., Safety and efficacy of intravenous glyburide on brain swelling after large hemispheric infarction (GAMES-RP): a randomised, double-blind, placebo-controlled phase 2 trial, Lancet Neurol, 15:1160-9, PMID 27567243 (2016)).

Preclinical work has shown that glibenclamide administered via transdermal skin patch yields steady plasma levels of drug that can be maintained below the hypoglycemic threshold (Mutalik et al., Glibenclamide transdermal patches: physicochemical, pharmacodynamic, and pharmacokinetic evaluations, J Pharm Sci, 93:1577-94, PMID 15124215 (2004); Anitha et al., Preparation, in-vitro and in-vivo characterization of transdermal patch containing glibenclamide and atenolol: a combinational approach, Pak J Pharm Sci, 24:155-63, PMID 21454164 (2011)). Thus, transdermal patches of glibenclamide could be used in patients with neuropathic pain. However, some side effects of glibenclamide have been reported, with some diabetic patients experiencing diarrhea, dizziness, headache, heartburn, nausea, gas and weight gain, in addition to altered glucose levels. Such side effects may not be well tolerated in some patients. In these patients, an alternative administration route can be intrathecal glibenclamide, e.g., administered via implanted pump. Our pilot data demonstrate that intrathecal treatment for suppression of SUR1 is highly effective at reducing allodynia. Intrathecal glibenclamide administration using an FDA-approved implanted pump (Medtronic^{®}) is now standard-of-care for baclofen, used in the treatment of spasticity. Recently, the FDA approved Medtronic's personal therapy manager (*myPTM*), which works with the company's *SynchroMed II* intrathecal drug delivery system for patients who suffer from chronic pain. The Medtronic SynchroMed II pump and catheter are implanted under the skin and deliver pain medication into the intrathecal space. Instead of analgesics, such a system can be used to deliver an injectable formulation of glibenclamide.

Thus, in accordance with the methods of the invention set forth herein, glibenclamide administered via transdermal skin patches, glibenclamide administered intrathecally, and/or glibenclamide administered by other routes of administration as described herein or as would be understood by a person of ordinary skill in the art, can be used to treat patients having neuropathic pain. Importantly, unlike opioids, glibenclamide does not modify normal mechanical or thermal sensitivity, but exerts antihypersensitivity (antiallodynic) effects only in sensitizing conditions.

### EXAMPLE 3 - General Methods.

***Survival surgery to expose the sciatic nerve.*** Survival surgeries are performed under anesthesia using aseptic/sterile conditions by scientists experienced in laboratory animal survival surgery. A mouse is anaesthetized (ketamine/xylazine), hair is clipped from the right proximal lateral thigh, and the skin is prepped with Betadine solution. Using a surgical microscope, the common branch of the right sciatic nerve is exposed by separating the muscles. For the sciatic n. cuff model of neuropathic pain, a 2 mm long section of pre-split PE-20 tubing is placed around the nerve (See Fig. 9) (Yalcin , et al., The sciatic nerve cuffing model of neuropathic pain in mice, J Vis Exp, PMID 25078668 (2014)).

***For nerve-specific downregulation of SUR1 in glia,*** a drug-eluting perineural device, preloaded with endoxifen, is placed loosely around the sciatic n. of *Abcc8*^{loxP/loxP}/*GFAP*-Cre/ERT2 mice to administer endoxifen locally (Zadelaar et al., Local Cre-mediated gene recombination in vascular smooth muscle cells in mice, Transgenic Res, 15:31-6, PMID 16475008 (2006); Benedykcinska et al., Generation of brain tumours in mice by Cre-mediated recombination of neural progenitors in situ with the tamoxifen metabolite endoxifen, Dis Model Mech,;9:211-20, PMID 26704996 (2016)), directly on the nerve for 5 days, 30 days before nerve cuffing. Endoxifen (Sigma) is first blended with polyethylene glycol (**PEG**) and the blend is mixed with molten poly(ε-caprolactone) (**PCL**) at 70 °C. The PCL:PEG ratio is 4:1 (w/w). Drug-loaded devices are made from the molten endoxifen-polymer mixture and designed to fit around the sciatic n. of mice. Drug-eluting devices have the shape of a longitudinal cut cylinder with an internal diameter of 0.6 mm, an external diameter of 1 mm, a length of 2 mm, and a weight of ~5 mg. Devices are loaded with 1% (w/w) endoxifen. Following surgery, incisions are closed with interrupted 5-0 nylon sutures. The mouse is recovered from anesthesia on a warming blanket.

***Spinal cord-specific inhibition or downregulation of SUR1 in central glia.*** The surgical technique for glibenclamide (in WT mice) or endoxifen (in *Abcc8*^{loxP/loxP}/*GFAP-*Cre/ERT2mice) infusion into the thecal sac is adapted from methods we use for spinal cord injury (Hosier et al., A Direct Comparison of Three Clinically Relevant Treatments in a Rat Model of Cervical Spinal Cord Injury, J Neurotrauma, 32:1633-44, PMID 26192071 (2015)). The anesthetized mouse is mounted in a stereotaxic apparatus, hair is clipped from the dorsal thoracolumbar region, and the skin is prepped with Betadine solution. Using microscopic visualization, a midline incision is made, the paraspinal muscles are dissected off of the spinous processes and laminae. The spinous processes of L6-S1 are removed using a high speed surgical drill, and the dura between them is punctured using a 27-g needle. The infusion cannula of a Brain infusion kit (30 gauge cannula; Brain Infusion kit 3) is advanced stereotactically 1 mm into the thecal sac and is secured to the spine with cement. The infusion cannula is connected to a mini-osmotic pump (#2002; 0.5 µL/hr; Alzet) for infusion of glibenclamide (3-30 ng/hr × 2 weeks) or endoxifen (5 mM in 40% DMSO × 5 days).Pumps are removed surgically at the indicated times.

***Behavioral Tests.*** Animals are handled 2-3× weekly for acclimatization to handlers. Motor and sensory testing is performed before surgery, and every 5 days thereafter. Behavioral results from post-surgery time points are normalized to baseline levels. Tests for hypersensitivity are performed as in our preliminary data.
*MECHANICAL ALLODYNIA.* The threshold for ipsi- and contralateral hindpaw withdrawal is determined using von Frey filaments, von Frey filaments are applied to the plantar surface of each hindpaw (0.16-4 gm). The "up-down method (Chaplan et al., Quantitative assessment of tactile allodynia in the rat paw, J Neurosci Methods, 53:55-63, PMID 7990513 (1994))" is used to determine the withdrawal threshold, as in our previous studies (Masri et al., Zona incerta: a role in central pain, J Neurophysiol, 102:181-91, PMID 19403748 (2009); Whitt et al., Pathological activity in mediodorsal thalamus of rats with spinal cord injury pain, J Neurosci, 33:3915-26, PMID 23447602 (2013)). Alternatively, the withdrawal threshold is determined using the Hugo Basile Dynamic Plantar Aesthesiometer (Stoelting).
*THERMAL ALLODYNIA* is assessed using acetone (Carey et al., Inflammatory and Neuropathic Nociception is Preserved in GPR55 Knockout Mice, Sci Rep, 7:944, PMID 28428628 (2017)). A 10 µL volume of acetone is propelled via air burst from a 200 µL pipette onto the mid-plantar surface of the hind paw without touching the skin. Time spent elevating/licking/biting/shaking the stimulated paw, up to 15 sec, is measured with a stop watch. Each paw is stimulated 5×, allowing 5 min to elapse between successive paw stimulations.
*THERMAL HYPERALGESIA* is assessed using the Hargreaves method, using a Hargreaves-type apparatus (Ugo Basile Plantar Test Instrument; Stoelting). An unrestrained animal is placed in a Perspex enclosure on the top of glass pane. An infrared generator placed below glass pane is aimed at the plantar surface of the hind paw and the time to withdrawal is recorded automatically via an optic sensor, avoiding experimental bias. Paw-withdrawal latency is calculated as the mean of 3-5 different measurements taken at 15 min intervals.
*DYNAMIC MECHANICAL ALLODYNIA* (**DMA**) measures neuropathic pain in humans and animals, mediated by Aβ fibers (Duan et al., Identification of spinal circuits transmitting and gating mechanical pain, Cell, 159:1417-32, PMID 25467445 (2014)). Its diagnostic advantage stems from the correlation between DMA scores and patient reports of ongoing pain, and from evidence that its expression involves central mechanisms. DMA is assessed by stimulating the lateral plantar region of the hindpaw with light stroking from heel to toe with a soft brush (Duan et al., Identification of spinal circuits transmitting and gating mechanical pain, Cell, 159:1417-32, PMID 25467445 (2014)).
*HINDLIMB MOTOR FUNCTION* is assessed using the accelerating rotarod. The mouse is placed on the rod rotating at 4 rpm. After 10 sec, rotation is accelerated at a rate of 20 rpm/min. The mean speed at fall-off is the datum.
*HINDLIMB MOTOR FUNCTION* is also assessed using foot misplacement. The Foot Misplacement Apparatus (Columbus Instruments) consists of a stainless steel horizontal ladder positioned over a plate. After the animal is conditioned, testing is performed by placing the animal on one end of the ladder, and counting the number of missteps as it moves toward a dark compartment at the other end of the ladder. Counting missteps is done automatically by detecting the change of resistance between the ladder and the metal plate.
*ANXIETY-LIKE* and *DEPRESSION-LIKE BEHAVIORS* are assessed only one time, they are administered during the 3 days preceding euthanasia, and the order in which tests are applied is from least stressful to more stressful (as listed below). We follow standard protocols, fully described in Yalcin et al (Yalcin et al., A time-dependent history of mood disorders in a murine model of neuropathic pain, Biol Psychiatry, 70:946-53, PMID 21890110 (2011)).
*Open field testing* is employed for measuring locomotor activity and willingness to explore in mice. A computerized tracking system (ANY-Maze; Stoelting Co.) records animal movements and calculates speed, distance and other parameters. Time spent exploring the center of the open field is inversely related to anxiety.
*Elevated Plus Maze* (Colbourn Instruments). Rodents naturally tend to explore novel environments, but open, well lit, elevated areas are perceived as aversive. This test is widely used in rodent models of anxiety.
*Tail suspension test* (Harvard Apparatus), which measures immobility time, as well as energy and power in motion, is employed as a measure of depression-like behavior in rodents.
*Porsolt forced swim test* is used for rodent models of depression. Behaviors are recorded using the Forced SwimScan system (Cleversys Inc.), which analyzes immobility, swimming, and escape behaviors.

***Tissue analysis.*** Spinal cords and sciatic nerves are perfusion-fixed (4% PFA), cryoprotected and cryosectioned (7 µm). Sciatic nerves within PE-20 cuffs are examined (H&E) to ensure equivalent injury. The DRG and dorsal horns of L3-4, which innervate the plantar surface of the hindpaw (Rigaud et al., Species and strain differences in rodent sciatic nerve anatomy: implications for studies of neuropathic pain, Pain, 136:188-201, PMID 18316160 (2008)), are examined to quantify changes in, and the time course of those changes, for markers of astrocyte activation (GFAP), microglia/macrophage activation (Iba1, CD68), SUR1-TRPM4, IL-6, the chemokines, CCL2 and CXCL1 and their cognate receptors, CCR2 and CXCR2. Expression of these markers will be quantitatively compared in the different treatment groups using immunohistochemistry and in situ hybridization with cell-specific markers (GFAP, NeuN, Iba1, CD45). Quantitative comparisons will be validated using immunoblot and qPCR.
*Immunohistochemistry and in situ hybridization.* Tissues are immunolabeled using commercial primary antibodies, as we described (Kurland et al., The Sur1-Trpm4 channel regulates NOS2 transcription in TLR4-activated microglia, J Neuroinflammation, 13:130, PMID 27246103 (2016)), with cell-specific co-labeling used to document involvement by individual cell types, with special attention directed to evaluating astrocytes and neurons. Sections are processed for in situ hybridization, with the same section also immunolabeled for the same protein, as we described (Mehta et al., Sur1-Trpm4 Cation Channel Expression in Human Cerebral Infarcts, J Neuropathol Exp Neurol, 74:835-49, PMID 26172285 (2015)). Fluorescent secondary antibodies are species-appropriate Alexa Fluor 488 or 555. Omission of primary antibody and competition with antigenic peptides are used as negative controls.
*Immuno-FRET (Förster Resonance Energy Transfer).* Immuno-FRET, which is used to determine heteromeric co-assembly of SUR1 and TRPM4, is performed as we described (Mehta et al., Sur1-Trpm4 Cation Channel Expression in Human Cerebral Infarcts, J Neuropathol Exp Neurol, 74:835-49, PMID 26172285 (2015)).
*Immunoblot and qPCR.* Tissues are flash-frozen and processed as we reported (Patel et al., Glibenclamide reduces hippocampal injury and preserves rapid spatial learning in a model of traumatic brain injury, J Neuropathol Exp Neurol, 69:1177-90, PMID 21107131 (2010)), and as shown in preliminary data (Fig. 5). Tissues are processed for immunoblot and qPCR as we described (Kurland et al., The Sur1-Trpm4 channel regulates NOS2 transcription in TLR4-activated microglia, J Neuroinflammation, 13:130, PMID 27246103 (2016); Patel et al., Glibenclamide reduces hippocampal injury and preserves rapid spatial learning in a model of traumatic brain injury, J Neuropathol Exp Neurol, 69:1177-90, PMID 21107131 (2010).
*Stereology* (Mandarim-de-Eacerda et al., Image analysis and quantitative morphology, Methods Mol Biol, 611:211-25, PMID 19960334 (2010)). Sections are immunolabeled in a single batch, and all images for a given marker are captured using uniform parameters of magnification, area, exposure, and gain, as we described (Patel et al., Glibenclamide reduces hippocampal injury and preserves rapid spatial learning in a model of traumatic brain injury, J Neuropathol Exp Neurol, 69:1177-90, PMID 21107131 (2010)). Labeled cells are counted using the optical fractionator protocol of the Stereologer (Stereology Resources Center, Chester MD).

***Data analysis.*** The Shapiro- Wilk' s test and inverse probability plots are used to test whether data are normally distributed. To compare values in different groups, a 1-way Analysis of Variance (**ANOVA**) or the non-parametric Kruskal-Wallis 1-way ANOVA by ranks is used. Multiple pairwise comparisons are conducted with Tukey's test for ANOVA or Nemenyi's test for nonparametric ranked data.

### Mice

Wild-type (WT), and *Abcc8*^{loxP/loxP}/*GFAP*-Cre/ERT2, all C57BL/6 background, both males and females ~25-30 gm, are used in the experiments described herein.

WT C57BL/6J mice, and GFAP-Cre/ERT2 mice (B6.Cg-Tg(GFAP-cre/ERT2)505Fmv/J; stock number 012849) are obtained from The Jackson Laboratory (Bar Harbor, ME).

*Abcc8*-floxed mice were generated and characterized in detail by Bryan and colleagues (Nakamura et al., Targeting SUR1/Abcc8-type neuroendocrine KATP Channels in Pancreatic Islet Cells, PLoS One, 9:e91525, PMID 24621811 (2014); Seghers et al., Sur1 knockout mice. A model for K(ATP) channel-independent regulation of insulin secretion, J Biol Chem, 275:9270-7, PMID 10734066 (2000), who generously provided us with breeders.

Tamoxifen-inducible *Abcc8*^{loxP/loxP}/*GFAP-*Cre/ERT2 knock-out mice are produced by crossing homozygous *Abcc8*-floxed mice with heterozygous tamoxifen-inducible GFAP/CreERT2 mice in which the Cre recombinase, fused to a mutated estrogen ligand-binding domain (ERT2) that requires the presence of tamoxifen for activity, is under the control of the GFAP promoter.

### Description of Procedures

***Survival surgery to expose the sciatic nerve.*** Survival surgeries are performed under anesthesia using aseptic/sterile conditions by scientists experienced in laboratory animal survival surgery. A mouse is anaesthetized (ketamine/xylazine), hair is clipped from the right proximal lateral thigh, and the skin is prepped with Betadine solution. Using a surgical microscope, the common branch of the right sciatic nerve is exposed by separating the muscles. For the sciatic nerve cuff model of neuropathic pain, a 2 mm long section of pre-split PE-20 tubing is placed around the nerve (see Fig. 9) (Yalcin , et al., The sciatic nerve cuffing model of neuropathic pain in mice, J Vis Exp, PMID 25078668 (2014)).

***For nerve-specific downregulation of SUR1 in glia,*** a drug-eluting perineural device, preloaded with endoxifen, is placed loosely around the sciatic n. of *Abcc8*^{loxP/loxP}/*GFAP*-Cre/ERT2 mice to administer endoxifen locally (Zadelaar et al., Local Cre-mediated gene recombination in vascular smooth muscle cells in mice, Transgenic Res, 15:31-6, PMID 16475008 (2006); Benedykcinska et al., Generation of brain tumours in mice by Cre-mediated recombination of neural progenitors in situ with the tamoxifen metabolite endoxifen, Dis Model Mech,;9:211-20, PMID 26704996 (2016)), directly on the nerve for 7 days before nerve cuffing. In brief, endoxifen (Sigma) is first blended with PEG before this blend is mixed with molten poly(ε-caprolactone) (**PCL**) at 70 °C. The PCL:PEG ratio is 4:1 (w/w). Drug-loaded devices are made from the blended molten endoxifen-polymer mixture and designed to fit around the sciatic n. of mice. Drug-eluting devices have the shape of a longitudinal cut cylinder with an internal diameter of 0.6 mm, an external diameter of 1 mm, a length of 2 mm, and a weight of approximately 5 mg. Devices are loaded with 1% (w/w) endoxifen. Following surgery, incisions are closed with interrupted 5-0 nylon sutures. The mouse is recovered from anesthesia on a warming blanket, then is returned to its cage.

***Spinal cord-specific downregulation of SUR1 in glia.*** The surgical techniques for endoxifen infusion into the thecal sac is adapted from the methods we use in this laboratory for spinal cord injury (Hosier et al., A Direct Comparison of Three Clinically Relevant Treatments in a Rat Model of Cervical Spinal Cord Injury, J Neurotrauma, 32:1633-44, PMID 26192071 (2015)). The anesthetized mouse is mounted in a stereotaxic apparatus, hair is clipped from the dorsal thoracolumbar region, and the skin is prepped with Betadine solution. Using a midline incision, the paraspinal muscles are dissected off of the spinous processes and laminae. The spinous processes of L6-S1 are removed using a high speed surgical drill, and the dura between them is punctured using a 27-g needle. The infusion cannula of a Brain infusion kit (30 gauge cannula; Brain Infusion kit 3) is advanced stereotactically 1 mm into the thecal sac and is secured in place with cement. The infusion cannula is connected to a mini-osmotic pump (#2001; 1.0 µL/hr; Alzet), which is implanted in a subcutaneous pocket on the dorsal thorax, for infusion of endoxifen (5 mM in 40% DMSO) × 7 days.

***Behavioral Tests.*** Animals are handled 2-3× weekly for acclimatization to handlers. Motor and sensory testing is performed before surgery, and every 5 days thereafter. Behavioral results from post-surgery time points are normalized to baseline levels. Tests for hypersensitivity are performed as in our preliminary data.
*MECHANICAL ALLODYNIA.* The threshold for ipsi- and contralateral hindpaw withdrawal is determined using von Frey filaments, von Frey filaments are applied to the plantar surface of each hindpaw (0.16-4 gm). The "up-down method (Chaplan et al., Quantitative assessment of tactile allodynia in the rat paw, J Neurosci Methods, 53:55-63, PMID 7990513 (1994))" is used to determine the withdrawal threshold, as in our previous studies (Masri et al., Zona incerta: a role in central pain, J Neurophysiol, 102:181-91, PMID 19403748 (2009); Whitt et al., Pathological activity in mediodorsal thalamus of rats with spinal cord injury pain, J Neurosci, 33:3915-26, PMID 23447602 (2013)). Alternatively, the withdrawal threshold is determined using the Hugo Basile Dynamic Plantar Aesthesiometer (Stoelting).
*THERMAL ALLODYNIA* is assessed using acetone (Carey et al., Inflammatory and Neuropathic Nociception is Preserved in GPR55 Knockout Mice, Sci Rep, 7:944, PMID 28428628 (2017)). A 10 µL volume of acetone is propelled via air burst from a 200 µL pipette onto the mid-plantar surface of the hind paw without touching the skin. Time spent elevating/licking/biting/shaking the stimulated paw, up to 15 sec, is measured with a stop watch. Each paw is stimulated 5×, allowing 5 min to elapse between successive paw stimulations.
*THERMAL HYPERALGESIA* is assessed using the Hargreaves method, using a Hargreaves-type apparatus (Ugo Basile Plantar Test Instrument; Stoelting). An unrestrained animal is placed in a Perspex enclosure on the top of glass pane. An infrared generator placed below glass pane is aimed at the plantar surface of the hind paw and the time to withdrawal is recorded automatically via an optic sensor, avoiding experimental bias. Paw-withdrawal latency is calculated as the mean of 3-5 different measurements taken at 15 min intervals.
*DYNAMIC MECHANICAL ALLODYNIA* (**DMA**) measures neuropathic pain in humans and animals, mediated by Aβ fibers (Duan et al., Identification of spinal circuits transmitting and gating mechanical pain, Cell, 159:1417-32, PMID 25467445 (2014)). Its diagnostic advantage stems from the correlation between DMA scores and patient reports of ongoing pain, and from evidence that its expression involves central mechanisms. DMA is assessed by stimulating the lateral plantar region of the hindpaw with light stroking from heel to toe with a soft brush (Duan et al., Identification of spinal circuits transmitting and gating mechanical pain, Cell, 159:1417-32, PMID 25467445 (2014)).
*HINDLIMB MOTOR FUNCTION* is assessed using the accelerating rotarod. The mouse is placed on the rod rotating at 4 rpm. After 10 sec, rotation is accelerated at a rate of 20 rpm/min. The mean speed at fall-off is the datum.
*HINDLIMB MOTOR FUNCTION* is also assessed using foot misplacement. The Foot Misplacement Apparatus (Columbus Instruments) consists of a stainless steel horizontal ladder positioned over a plate. After the animal is conditioned, testing is performed by placing the animal on one end of the ladder, and counting the number of missteps as it moves toward a dark compartment at the other end of the ladder. Counting missteps is done automatically by detecting the change of resistance between the ladder and the metal plate.
*ANXIETY-LIKE* and *DEPRESSION-LIKE BEHAVIORS* are assessed only one time, they are administered during the 3 days preceding euthanasia, and the order in which tests are applied is from least stressful to more stressful (as listed below). We follow standard protocols, fully described in Yalcin et al (Yalcin et al., A time-dependent history of mood disorders in a murine model of neuropathic pain, Biol Psychiatry, 70:946-53, PMID 21890110 (2011)).
*Open field testing* is employed for measuring locomotor activity and willingness to explore in mice. A computerized tracking system (ANY-Maze; Stoelting Co.) records animal movements and calculates speed, distance and other parameters. Time spent exploring the center of the open field is inversely related to anxiety.
*Elevated Plus Maze* (Colboum Instruments). Rodents naturally tend to explore novel environments, but open, well lit, elevated areas are perceived as aversive. This test is widely used in rodent models of anxiety.
*Tail suspension test* (Harvard Apparatus), which measures immobility time, as well as energy and power in motion, is employed as a measure of depression-like behavior in rodents.
*Por saltforced swim test* is used for rodent models of depression. Behaviors are recorded using the Forced SwimScan system (Cleversys Inc.), which analyzes immobility, swimming, and escape behaviors.

EXAMPLE 4 - Various formulations and routes of administration of glibenclamide are effective at ameliorating neuropathic pain behaviors following peripheral nerve injury.

On day -1, adult male mice were assessed for baseline tactile (von Frey filaments) and thermal (Hargreaves plantar test; IITC Inc. Life Sciences) sensation, and exhibited normal function. On day 0, all mice underwent unilateral sciatic nerve cuffing, in which a 2 mm long section of pre-split PE-20 tubing is placed around the right sciatic nerve under sterile conditions (Yalcin et al., The sciatic nerve cuffing model of neuropathic pain in mice, J Vis Exp, PMID 25078668 (2014)). On day +14, repeat testing of tactile (von Frey filaments) and thermal (Hargreaves method) sensation revealed significant neuropathic pain behaviors, including mechanical allodynia, and thermal hyperalgesia. On day +15, mice were randomly assigned to one of three groups (5 mice/group): [1] placebo; [2] slow-release glibenclamide pellets implanted subcutaneously (pellets release glibenclamide, 12 µg/day ×21 days; catalogue #G-101; Innovative Research of America); [3] intrathecal glibenclamide, delivered at lumbar 5, using an implanted Alzet Brain infusion kit and mini-osmotic pump (#2002), delivering glibenclamide 5 ng/hour (0.5 µL/hour) ×14 days. On day +28, repeat testing of tactile (von Frey filaments) and thermal (Hargreaves method) sensation revealed significant amelioration of neuropathic pain behaviors: the slow release subcutaneous formulation partially reversed mechanical allodynia and completely reversed thermal hyperalgesia, while intrathecal glibenclamide completely reversed both mechanical allodynia and thermal hyperalgesia. See Fig. 17.

## Claims

1. Agent that inhibits the activity of an NC_{Ca-ATP} channel for the use of treating neuropathic pain in a subject in need thereof, by administering to the subject an effective amount of said agent, wherein the agent is a) a SUR1 antagonist; or b) a Transient Receptor Potential cation channel subfamily M member 4 (TRPM4) antagonist,
wherein the SUR1 antagonist is selected from the group consisting of glibenclamide (glyburide), tolbutamide, acetohexamide, chlorpropamide, tolazimide, glipizide, gliquidone, repaglinide, nateglinide, meglitinide, gliclazide, glimepiride, repaglinide, nateglinide, and mitiglinide, and
wherein the TRPM4 antagonist is selected from flufenamic acid, mefanimic acid, and niflumic acid.

2. Agent for the use of claim 1, wherein the agent is administered by intravenous, subcutaneous, intramuscular, intracutaneous, transcutaneous, intragastric or oral administration.

3. Agent for the use of claims 1 or 2, wherein the agent is administered to the subject at a dosage of less than 500 mg per day.

4. Agent for the use of any of claims 1-3, wherein the agent is administered to the subject at a dosage of less than 0.8 mg/kg body weight within a 24 hour period.

5. Agent for the use of any of claims 1-4, wherein the method further comprises administering an additional therapeutic agent to the subject to treat pain.

6. Agent for the use of any of claims 1-5, wherein the agent that inhibits the activity of an NC_{Ca-ATP} channel is glibenclamide or tolbutamide.

7. Agent for the use of any of claims 1-6, wherein the amount of the agent that inhibits the activity of an NC_{Ca-ATP} channel is administered to the subject is in the range of about 0.0001 µg/kg/day to about 20 mg/kg/day, preferably in the range of about 0.01 µg/kg/day to about 100 µg/kg/day, more preferably in the range of about 100 µg/kg/day to about 20 mg/kg/day.

8. Agent for the use of any of claims 1-7, wherein the agent that inhibits the activity of an NC_{Ca-ATP} channel is administered as a bolus injection.

9. Agent for the use of any of claims 1-8, wherein the agent that inhibits the activity of an NC_{Ca-ATP} channel is administered as an infusion.

10. Agent for the use of any of claims 1-9, wherein the agent that inhibits the activity of an NC_{Ca-ATP} channel is administered as a bolus injection in combination with an infusion.

11. Agent for the use of any of claims 1-10, wherein the agent is administered as a loading dose followed by a constant infusion.

12. Agent for the use of any of claims 1-11, wherein the subject suffers from peripheral nerve injury.

## Patentansprüche

1. Mittel, das die Aktivität eines NC_{Ca-ATP}-Kanals hemmt, zur Verwendung bei der Behandlung von neuropathischem Schmerz bei einem Individuum, das dies benötigt, durch Verabreichung einer wirksamen Menge des Mittels an das Individuum, wobei das Mittel a) ein SUR1-Antagonist; oder b) ein Antagonist des Transient-Receptor-Potential-Kationenkanal-Unterfamilie-M-Mitglieds 4 (TRPM4) ist,
wobei der SUR1-Antagonist aus der aus Glibenclamid (Glyburid), Tolbutamid, Acetohexamid, Chlorpropamid, Tolazimid, Glipizid, Gliquidon, Repaglinid, Nateglinid, Meglitinid, Gliclazid, Glimepirid, Repaglinid, Nateglinid und Mitiglinid bestehenden Gruppe ausgewählt ist und
wobei der TRPM4-Antagonist aus Flufenaminsäure, Mefenaminsäure und Nifluminsäure ausgewählt ist.

2. Mittel zur Verwendung nach Anspruch 1, wobei das Mittel durch intravenöse, subkutane, intramuskuläre, intrakutane, transkutane, intragastrische oder orale Verabreichung verabreicht wird.

3. Mittel zur Verwendung nach Anspruch 1 oder 2, wobei das Mittel dem Individuum in einer Dosis von weniger als 500 mg pro Tag verabreicht wird.

4. Mittel zur Verwendung nach einem der Ansprüche 1-3, wobei das Mittel dem Individuum in einer Dosis von weniger als 0,8 mg/kg Körpergewicht innerhalb eines Zeitraums von 24 Stunden verabreicht wird.

5. Mittel zur Verwendung nach einem der Ansprüche 1-4, wobei das Verfahren weiters die Verabreichung eines zusätzlichen Therapeutikums an das Individuum umfasst, um Schmerz zu behandeln.

6. Mittel zur Verwendung nach einem der Ansprüche 1-5, wobei das Mittel, das die Aktivität eines NC_{Ca-ATP}-Kanals hemmt, Glibenclamid oder Tolbutamid ist.

7. Mittel zur Verwendung nach einem der Ansprüche 1-6, wobei die dem Individuum verabreichte Menge des Mittels, das die Aktivität eines NC_{Ca-ATP}-Kanals hemmt, im Bereich von etwa 0,0001 µg/kg/Tag bis etwa 20 mg/kg/Tag liegt, vorzugsweise im Bereich von etwa 0,01 µg/kg/Tag bis etwa 100 µg/kg/Tag, noch bevorzugter im Bereich von etwa 100 µg/kg/Tag bis etwa 20 mg/kg/Tag.

8. Mittel zur Verwendung nach einem der Ansprüche 1-7, wobei das Mittel, das die Aktivität eines NC_{Ca-ATP}-Kanals hemmt, als Bolusinjektion verabreicht wird.

9. Mittel zur Verwendung nach einem der Ansprüche 1-8, wobei das Mittel, das die Aktivität eines NC_{Ca-ATP}-Kanals hemmt, als Infusion verabreicht wird.

10. Mittel zur Verwendung nach einem der Ansprüche 1-9, wobei das Mittel, das die Aktivität eines NC_{Ca-ATP}-Kanals hemmt, als Bolusinjektion in Kombination mit einer Infusion verabreicht wird.

11. Mittel zur Verwendung nach einem der Ansprüche 1-10, wobei das Mittel als Aufsättigungsdosis verabreicht wird.

12. Mittel zur Verwendung nach einem der Ansprüche 1-11, wobei das Individuum an einer peripheren Nervenverletzung leidet.

## Revendications

1. Agent qui inhibe l'activité d'un canal NC_{Ca_ATP} à utiliser dans le traitement de la douleur neuropathique chez un sujet qui en a besoin, en administrant au sujet une quantité efficace dudit agent, dans lequel l'agent est a) un antagoniste de SUR1 ; ou b) un antagoniste du canal cationique potentiel de récepteur transitoire de la sous-famille M membre 4 (TRPM4),
dans lequel l'antagoniste de SUR1 est choisi dans le groupe consistant en glibenclamide (glyburide), tolbutamide, acétohexamide, chlorpropamide, tolazimide, glipizide, gliquidone, répaglinide, natéglinide, méglitinide, gliclazide, glimépiride, répaglinide, natéglinide et mitiglinide, et
dans lequel l'antagoniste de TRPM4 est choisi parmi l'acide flufénamique, l'acide méfanimique et l'acide niflumique.

2. Agent à utiliser selon la revendication 1, dans lequel l'agent est administré par voie intraveineuse, sous-cutanée, intramusculaire, intracutanée, transcutanée, intragastrique ou orale.

3. Agent à utiliser selon la revendication 1 ou 2, dans lequel l'agent est administré au sujet à une dose inférieure à 500 mg par jour.

4. Agent à utiliser selon l'une quelconque des revendications 1 à 3, dans lequel l'agent est administré au sujet à une dose inférieure à 0,8 mg/kg de poids corporel dans une période de 24 heures.

5. Agent à utiliser selon l'une quelconque des revendications 1 à 4, dans lequel le procédé comprend en outre l'administration d'un agent thérapeutique supplémentaire au sujet pour traiter la douleur.

6. Agent à utiliser selon l'une quelconque des revendications 1 à 5, dans lequel l'agent qui inhibe l'activité d'un canal NCca-ATp est le glibenclamide ou le tolbutamide.

7. Agent à utiliser selon l'une quelconque des revendications 1 à 6, dans lequel la quantité de l'agent qui inhibe l'activité d'un canal NC_{Ca-ATP} est administrée au sujet est dans la plage d'environ 0,0001 mg/kg/jour à environ 20 mg/kg/jour, de préférence dans la plage d'environ 0,01 mg/kg/jour à environ 100 mg/kg/jour, de manière plus préférée dans la plage d'environ 100 mg/kg/jour à environ 20 mg/kg/jour.

8. Agent à utiliser selon l'une quelconque des revendications 1 à 7, dans lequel l'agent qui inhibe l'activité d'un canal NC_{Ca-ATP} est administré sous la forme d'une injection en bolus.

9. Agent à utiliser selon l'une quelconque des revendications 1 à 8, dans lequel l'agent qui inhibe l'activité d'un canal NC_{Ca-ATP} est administré sous la forme d'une perfusion.

10. Agent à utiliser selon l'une quelconque des revendications 1 à 9, dans lequel l'agent qui inhibe l'activité d'un canal NC_{Ca-ATP} est administré sous la forme d'une injection en bolus en combinaison avec une perfusion.

11. Agent à utiliser selon l'une quelconque des revendications 1 à 10, dans lequel l'agent est administré en tant que dose de charge suivie d'une perfusion constante.

12. Agent à utiliser selon l'une quelconque des revendications 1 à 11, dans lequel le sujet souffre d'une lésion du nerf périphérique.
